# EUROPEAN PATENT APPLICATION

(11) **EP 3 769 741 A1**
(43) Date of publication of application: **27.01.2021**
(21) Application number: 20180784.9
(22) Date of filing: 18.06.2020
(51) Int. Cl.: A61H 35/00

(54) **LEG CARE APPARATUS**

(30) Priority: 24.07.2019 KR 20190089671; 24.07.2019 KR 20190089675; 24.07.2019 KR 20190089676; 24.07.2019 KR 20190089679; 24.07.2019 KR 20190089680; 24.07.2019 KR 20190089683; 24.07.2019 KR 20190089685; 24.07.2019 KR 20190089687; 24.07.2019 KR 20190089702
(71) Applicant: LG Electronics Inc., Yeongdeungpo-gu Seoul 07336 (KR)
(72) Inventor: YOO, Hyunsun, 08592 Seoul (KR); CHUN, Jaehung, 08592 Seoul (KR); KIM, Joogyeom, 08592 Seoul (KR)
(74) Representative: Vossius & Partner Patentanwälte Rechtsanwälte mbB

(57) **Abstract**

A leg care apparatus includes a main body configured to provide an action space in which a leg is accommodated and an atomizer configured to provide mist to the action space so as to care the leg disposed in the action space.

## Description

The present application claims priority under 35 U.S.C. 119 and 35 U.S.C. 365 to Korean Patent Application Nos. 10-2019-0089671, 10-2019-0089675, 10-2019-0089676, 10-2019-0089679, 10-2019-0089680, 10-2019-0089683, 10-2019-0089685, 10-2019-0089687, and 10-2019-0089702, filed on July 24, 2019, which is hereby incorporated by reference in its entirety.

The present disclosure relates to a leg care apparatus.

Foot bath is an action where user's feet are soaked in hot water for a predetermined time.

The foot bath is an action where heat is applied to the user's feet. In detail, the foot bath is known to be effective in improving various ailments such as blood circulation improvement, body temperature rise, improvement in feeling cold, improvement in sleep disorders, waste discharge, ingrown toenail prevention, plantar fasciitis improvement, stress relief, skin care, and the like by using heat transferred indirectly to the human body through the feet.

A device that provides hot water up to a height of the vicinity of an ankle to allow the user to soak their feet is widely known as a foot bath device that is capable of performing a foot bath. The foot bath device using water has limitations in that heat loss is large, the device is difficult to handle, and its use is troublesome because the feet have to be conduction-heated indirectly by heating water.

To solve these limitations, a foot bath device in which a heating element is provided inside a control space, and the foot bath is performed by using radiant heating using the heating element is being introduced. A foot bath machine using radiant heating is disclosed in Korean Patent registration No. 10-1145430.

The above-described device has the following limitations. First, there is inconvenience in that a user control panel is complicated to operate. Second, there is a limitation in that user's safety is threatened because a heating element is used. Third, there is a limitation in that the storage and movement of the device are difficult. Fourth, there is a limitation in that the device is frequently damaged due to having no rigidity. Fifth, it is troublesome to use because the control space is blocked by a plate. Sixth, there is a limitation in that only fomentation using the radiant heat is enabled in a radiant heating manner.

Embodiments provide a leg care apparatus that includes a foot bath device to care a leg.

Embodiments also provide a leg care apparatus which is conveniently operated and used by a user.

Embodiments also provide a leg care apparatus in which a temperature control state is safely applied to a user's leg.

Embodiments also provide a leg care apparatus that is conveniently moved and stored.

Embodiments also provide a leg care apparatus that is prevented from being damaged due to rigidity and is easy to be handled by a user.

Embodiments also provide a leg care apparatus that is capable of enjoying a foot bath in various manners.

In one embodiment, a leg care apparatus includes: a main body configured to provide an action space in which a leg is accommodated; and an atomizer configured to provide mist to the action space so as to care the leg disposed in the action space. According to an embodiment, foot bath may be performed by using the mist.

The leg care apparatus may further include a contact pad that is adjustable in temperature is exposed to an inner surface of the action space. The foot bath may be performed by a conductive method.

The contact pad may be disposed on an inner surface of the action space to correspond to at least one place of a user's calf or sole. Accordingly, the user may enjoy the foot bath by using a body part having high elasticity and a wide contact surface among portions of the leg.

One surface of the contact pad may correspond to a thermoelectric module to create a low or high temperature environment. Accordingly, the user may enjoy the foot bath safely and quickly in a conductive manner.

The leg care apparatus may further include: a bottom module which provides at least a portion of a bottom surface of the action space and in which components are accommodated therein; and a water storage part provided in a top surface of the bottom module to collect water. According to an embodiment, the foot bath may be performed using water collected in a bottom part of the action space.

A bottom surface of the water storage part may be inclined downward from a front side to a rear side so that the user conveniently places a sole, the foot is comfortable while being seated.

The atomizer may include a room-temperature atomizer configured to spray room-temperature mist. In this case, the user may conveniently enjoy a cold foot bath.

The atomizer may include a high-temperature atomizer configured to spray high-temperature mist. In this case, the user may conveniently enjoy a warm foot bath.

The room-temperature atomizer may conveniently supply the mist at room temperature by supplying fine water particles through ultrasonic waves.

The high-temperature atomizer may be operated in a tank heating manner in which water stored in a tank is heated to utilize high-temperature steam, thereby improving user's convenience.

The leg care apparatus may further include, to supply water to the atomizer,; an upper module that is relatively movable with respect to the main body; a water supply device disposed at one portion of the upper module to receive the water to be supplied to the atomizer; a water storage tank configured to supply the water to the atomizer and store the water received from the water supply device; and a conduct pipe configured to connect the water supply device to the water storage tank. Accordingly, the user may supply water from an upper side of the apparatus to conveniently supply the water and prevent the water from leaking, thereby preventing the apparatus from breaking down.

The conduct pipe may be expanded and contracted so that the water is stably supplied even if the action space adjustment module is moved, and the water may be managed.

The leg care apparatus may include a blower configured to generate air current within the action space. Accordingly, since energy is supplied to the mist covering the leg, the foot bath using the mist may be continuously performed for a predetermined time.

A heating wire configured to heat the air blown by the blower may be provided to provide hot air current directly from the blower.

The leg care apparatus may further include an action space adjustment module configured to adjust a volume of the action space, wherein the action space adjustment module may include at least one of: an upper module configured to adjust a vertical size of the action space; and a side module configured to adjust a front and rear size of the action space. Accordingly, the leg care apparatus may be opened upward to correspond to a front side, at which the user is placed, and a length of the user's leg, thereby improving user's convenience.

The leg care apparatus may further include a knee care part disposed to correspond to a user's knee within the action space. Accordingly, since a strong hot fomentation function is provided, the user's satisfaction may be improved. In particular, the user's satisfaction may be enhanced by strongly caring for the knee that is vulnerable to the elderly.

The knee care part may include: at least one light emitting element; and a massage pad configured to press or release a peripheral portion of the knee. Accordingly, not only the foot bath by heat may be performed, but also the pain of the knee may be alleviated by compression.

In another embodiment, a method for controlling a leg care apparatus includes: adjusting a size of an action space to fit a user's leg accommodated in an action space; and supplying fine water particles to cover the leg and controlling a temperature state of a contact pad for adjusting a contact temperature of a user's skin so as to care the user's leg. Accordingly, the leg may be cared by indirect convection and conduction.

To care the user's leg, the method may further include controlling a temperature state of the contact pad through which a contact temperature of a user's skin is adjusted. Accordingly, the heat and cold air may be directly transferred in the conductive manner to improve the user's satisfaction.

The fine water particles may be heated by hot air stream, and thus, the foot bath may be performed using the mist covering the leg even if the mist is not continuously supplied. Mist that is excessively supplied into the action space may be reduced. Furthermore, moisture supplied to an indoor space may be reduced, so that excessive moisture supply into the indoor space is prevented by the operation of the leg care device. As a result, the effect on the indoor space may be reduced.

The contact part may be controlled to be cooled so that a cold area is formed on an inner surface of the action space. In this case, the cold fomentation may be more strongly realized. Here, when the cold fine water particles are supplied together, the function of the cold fomentation function may be further enhanced.

The contact pad may correspond to any one of the calf and the sole of the user's foot, and thus, the user may enjoy the foot bath in various manners through the conductive heat transmitted to the skin.

In further another embodiment, a method for controlling a leg care apparatus includes: heating a contact pad contacting a user's skin within an action space in which a user's leg is accommodated; and allowing an action space adjustment module, which opens an inlet for inserting the user's leg into the action space, to be openable after the contact pad reaches a set temperature that is previously set. Accordingly, the user may enjoy the foot bath safely without inconvenience.

The details of one or more embodiments are set forth in the accompanying drawings and the description below. Other features will be apparent from the description and drawings, and from the claims.

### BRIEF DESCRIPTION OF THE DRAWINGS

FIGS. 1 and 2 are front perspective views of a leg care apparatus according to an embodiment, wherein FIG. 1 illustrates a state in which the leg care apparatus is stored, and FIG. 2 illustrates a state in which the leg care apparatus is operated.
FIG. 3 is a schematic cross-sectional view taken along line A-A' of FIG. 2.
FIG. 4 is an exploded side view of the entire leg care apparatus according to an embodiment.
FIG. 5 is an exploded side view of a main body.
FIG. 6 is an exploded side view of an upper module.
FIG. 7 is an exploded side view of a side module.
FIG. 8 is an exploded side view of a bottom module.
FIG. 9 is a side view of the leg care apparatus showing constituents related to an atomizer.
FIG. 10 is a rear view of the leg care apparatus showing the constituents related to the atomizer.
FIG. 11 is a schematic view of the atomizer.
FIG. 12 is a cross-sectional view illustrating an example of the atomizer.
FIG. 13 is a schematic view of a system related to the atomizer.
FIG. 14 is a schematic rear view of the leg care apparatus in which a room-temperature atomizer and a high-temperature atomizer are installed together.
FIG. 15 is an exploded side view illustrating a main body of a leg care apparatus according to another embodiment.
FIG. 16 is a flowchart for explaining a method for controlling a leg care apparatus according to an embodiment.
FIG. 17 is a flowchart for explaining a method for controlling a leg care apparatus according to another embodiment.
FIG. 18 is a flowchart for explaining a method for controlling a leg care apparatus according to another embodiment.
FIG. 19 is a view illustrating a configuration of the knee care part.
FIG. 20 is a flowchart for explaining a method for controlling a leg care apparatus according to another embodiment.
FIG. 21 is a flowchart for explaining a method for controlling a leg care apparatus according to another embodiment.
FIG. 22 is a schematic cross-sectional view that cuts a lower portion of the leg care apparatus in a front and rear direction according to another embodiment.
FIG. 23 is a top view of the leg care apparatus according to another embodiment.
FIG. 24 is a perspective view of a foot contact pad according to the another embodiment.
FIG. 25 is a perspective view of a foot contact pad according to another embodiment.

Hereinafter, exemplary embodiments will be described in detail with reference to the accompanying drawings. The invention may, however, be embodied in many different forms and should not be construed as being limited to the embodiments set forth herein, and a person of ordinary skill in the art, who understands the spirit of the present invention, may readily implement other embodiments included within the scope of the same concept by adding, changing, deleting, and adding components. Thus, it should be understood that they are also included within the scope of the present invention.

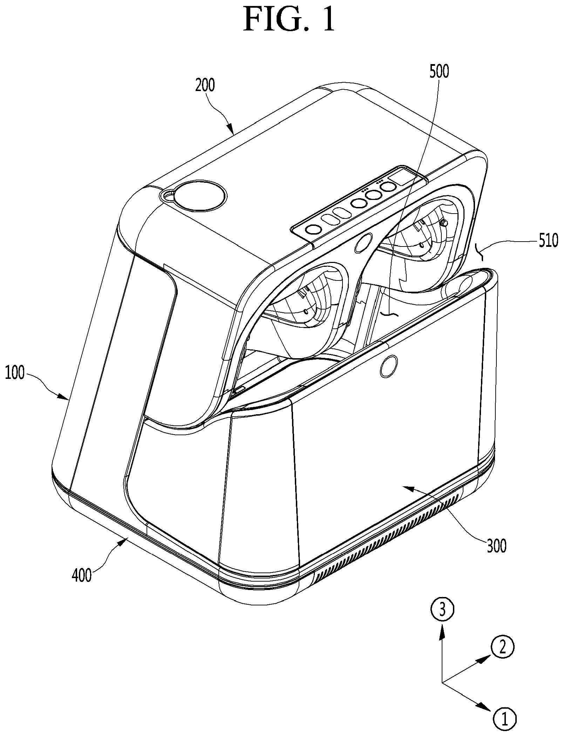

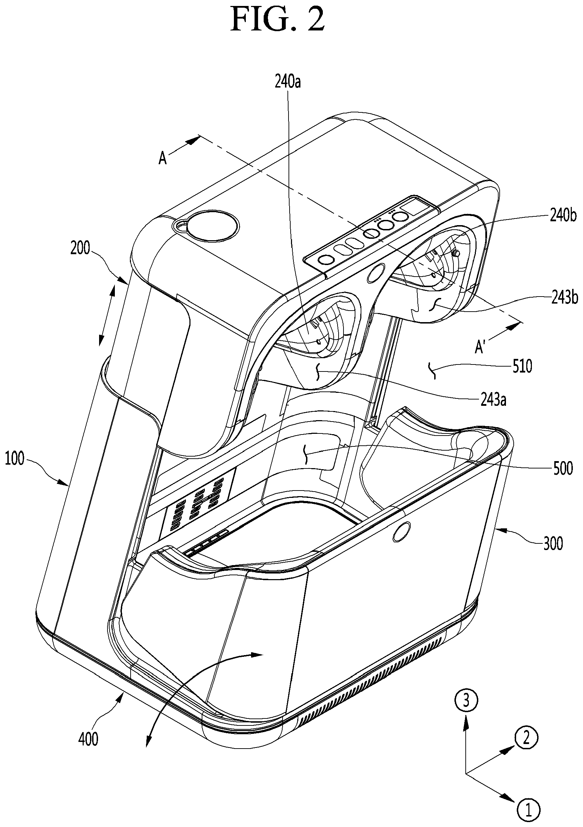

FIGS. 1 and 2 are front perspective views of a leg care apparatus according to an embodiment. That is, FIG. 1 illustrates a state in which the leg care apparatus is stored, and FIG. 2 illustrates a state in which the leg care apparatus is operated. Here, the storage state may mean a state in which the leg care apparatus has the smallest size or is not in use. The operation state may mean a state in which the leg care apparatus is expanded so that a user may insert their leg or a state in which the leg care apparatus is moved for use.

In the description of the drawings, a direction in which the user is accessed indicates a front side. When based on each axis shown in the figures, the front and rear direction is expressed as ①, and the direction in which the user accesses the leg care apparatus indicates the front side. A left and right direction is expressed as ② and indicates a left and right direction of the front side with respect to the user. An upward and downward direction is expressed as ③ and indicates an upward and downward direction of the front side with respect to the user.

In the leg care apparatus according to an embodiment, in order to allow the user's leg to be inserted, an inlet may increase in size, and an inner action space may increase in volume. After the user's leg is inserted, the inlet may decrease in size to be suitable for the user's body, and the action space may decrease in volume to be suitable for the user's leg. Since the action space and the inlet are adjusted to be suitable for a body size of the user, particularly, a size and length of the leg, a thermal effect acting on the leg may be largely and quickly applied, and energy consumption may be saved.

According to an embodiment, the leg may be cared for by applying hot or cold air and/or pressure to a leg portion including portions of knees, calves, and thighs together with the feet.

In the following description, the meaning of the foot bath not only means foot bath using water pressure and heat applied in the water, but also applying heat, cold air, and pressure to a leg portion including portions of feet, knees, calves, and thighs.

Referring to FIGS. 1 and 2, a leg care apparatus 10 according to an embodiment includes a main body 100, an upper module 200 connected to an upper portion of the main body 100 to largely open an upper space of the leg care apparatus 10, a side module 300 connected to a front portion of the main body 100 to largely open an inner space of the leg care apparatus 10, and a bottom module 400 connected to a lower portion of the main body 100 to accommodate components that are required for operation of the leg care apparatus 10.

An action space 500 is provided in an inner space inside an inner surface of each of the main body 100, the upper module 200, the side module 300, and the bottom module 400. The action space 500 is a space for applying hot or cold air to a user's leg through at least one manner of conduction, convection, or radiation. An inlet 510 through which the user's leg is inserted and withdrawn is provided in front of the action space 500. Since at least one of the hot or cold air is applied to the user's leg in at least one manner of the conduction, convection, or radiation, the user may have a foot bath in a manner selected from various manners that are desired by the user.

The upper module 200 may perform a vertical elevation operation.

When the upper module 200 is moved upward, the inlet 510 is largely opened so that the user may conveniently insert their leg into the action space 500. After the user inserts their leg into the action space 500, the upper module 200 may be moved downward. The upper module 200 may be moved downward until a portion of the user's leg touches or the action space 500 is constructed in a shape desired by the user. The upper module 200 may define at least a portion of a top surface of the action space.

The upper module 200 is provided to be slid vertically in the embodiment, but is not limited thereto. For example, the upper module 200 may be opened through a rotation operation or moved to a position desired by the user.

Knee care parts 240a and 240b, each of which having a recessed shape, may be provided at both left and right side at the front of the upper module 200. Inner surfaces of the knee care parts may be provided with knee placing parts 243a and 243b. Each of the knee placing parts is a portion that contacts the user's knee. The knee placing part may include a light emitting element and a pad. The light emitting element and the pad may apply at least one of heat or pressure to care the knee, thereby performing blood flow improvement, muscle stimulation, and pain improvement.

The knee care part 240 cares the knee by applying at least one of the heat or the pressure. The action space 500 cares the user's leg through conduction, convection, and radiation of the hot or cold air. According to an embodiment, the leg care apparatus may improve user's satisfaction by performing a suitable action for each location of the leg. Particularly, the action space 500 may function as a foot bath machine by performing a function of the foot bath, and the knee care part 240 may function as a knee massager. The leg care apparatus according to embodiment may perform at least the functions of the foot bath machine and the knee massager.

The side module 300 may perform the rotation operation forward and backward.

When the side module 300 rotates forward, the inlet 510 may be opened so that the user may conveniently insert their leg into the action space 500. After the user inserts the leg into the action space, the side module 300 may rotate backward. The side module 300 may rotate backwards until a portion of the user's leg touches, or the action space 500 is constructed in a shape desired by the user. The side module 300 may provide at least a portion of a front surface of the action space.

The side module 300 rotates backward and forward in this embodiment, but the embodiment is not limited thereto. For example, the side module 300 may be slid to be opened or adjusted to a position desired by the user.

As described above, in the leg care apparatus 10 according to an embodiment, the upper module 200 and the side module 300 are contracted when not in use. Accordingly, the leg care apparatus 10 may be easily stored, moved, and handled in a state of being contracted in volume.

The upper module 200 and the side module 300 may be operable with respect to the main body 100. As a result, the action space may increase or decrease in volume. Thus, the functions such as the convenient handling, the foot bath that is suitable for the user, the leg contact, and the like may be performed. The upper module 200 and the side module 300 may perform the action of adjusting the size and shape of the action space. Thus, the upper module 200 and the side module 300 may be referred to as action space adjustment modules.

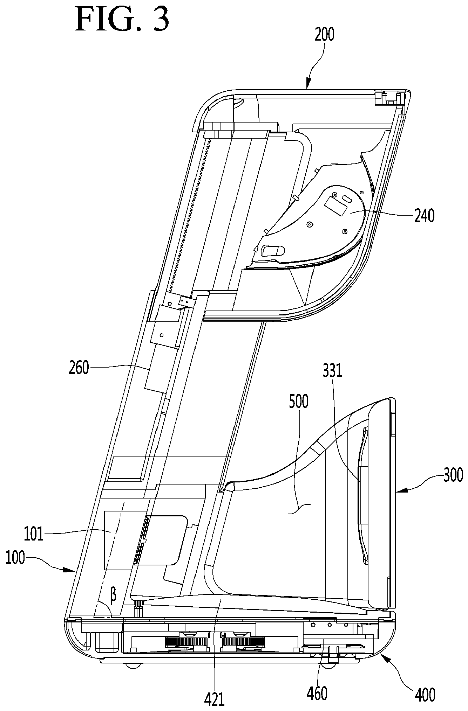

FIG. 3 is a schematic cross-sectional view taken along line A-A' of FIG. 2.

A schematic configuration and operation of the leg care apparatus according to an embodiment will be described with reference to FIG. 3. The main body 100 extends upward from a rear portion of the bottom module 400, and an upward extending angle is inclined forward at a predetermined angle β. Here, the inclined angle may be less than about 90 degrees as an acute angle. Since the main body 100 is inclined forward to extend, the user may not need to bend the knee excessively while inserting their leg into the action space 500 or while using the leg care apparatus.

Patients that need to care their leg by using the leg care apparatus may suffer from orthopedic diseases such as knee arthritis. The main body 100 may be provided to be inclined forward so that the action space 500 corresponds to a large bent angle of the user's leg without the patients having to excessively bend the knee. For example, the user may use the leg care apparatus even if the knee is not bent more than about 90 degrees.

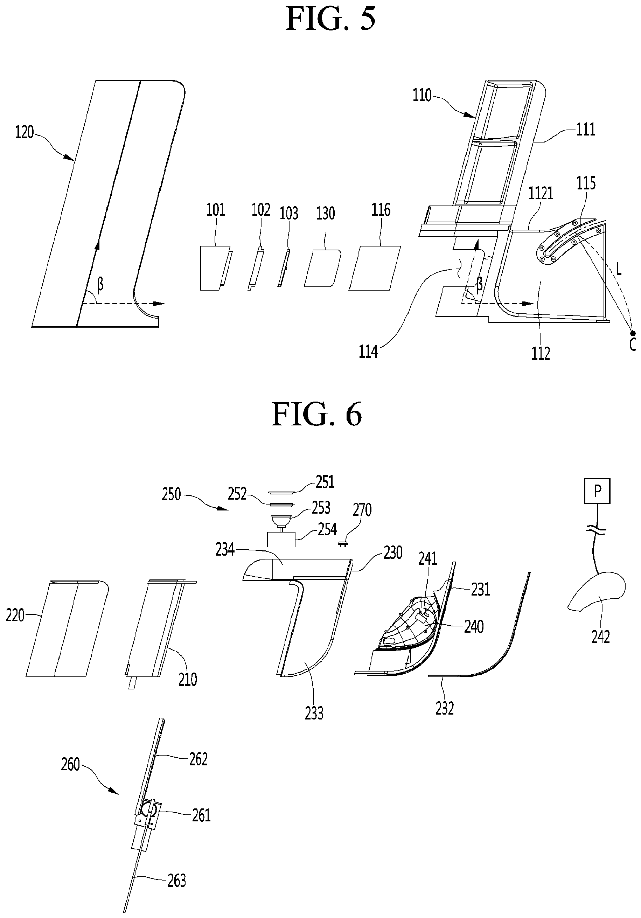

Since a main vertical extension part (see reference numeral 111 of FIG. 5) of the main body 100 is provided to be inclined forward, other components related thereto may also be provided to be inclined.

The upper module 200 is provided on an upper portion of the main body 100. A vertical opening device 260 may be inserted into a contact part between the upper module 200 and the main body 100. The vertical opening device 260 may include a driving motor and a gear train and may move the upper module 200 upward or downward with respect to the main body 100.

The upper module 200 being moved upward may be when the inlet 510 is opened so that the user's leg is inserted into the action space 500. Alternatively, the upper module 200 may be moved upward even when the user withdraws their leg from the action space 500. The upper module 200 being moved downward may be when the inlet 510 decreases in size, or the action space is contracted after the user inserts their leg.

The knee care part 240 may be disposed on a front portion of the upper module 200 to care the user's knee.

A blower 101 may be provided below the main body 100. The blower 101 may provide hot air into the action space 500. The hot air of the blower 101 may be heated by a heating wire provided in the blower 101. The blower 101 may perform an action for forced convection of air heated by an external separate heating device.

The bottom module 400 may be disposed on a bottom part of the leg care apparatus to support the entire apparatus at a lower side. A foot contact pad 421 may be disposed on a top surface of the bottom module 400. A sole of the foot may contact the foot contact pad 421. The foot contact pad 421 may perform a foot bath function by conducting a temperature atmosphere controlled by an external force to the user's foot.

A front and rear opening device 460 may be disposed on a front portion of the bottom module 400. The front and rear opening device 460 may include a motor and a gear train and may be inserted into a contact part between the bottom module 400 and the side module 300. The front and rear opening device 460 may move the side module 300 forward and backward with respect to the main body 100.

A calf contact pad 331 may be disposed on an inner surface of the side module 300. A user's calf may contact the calf contact pad 331. The calf contact pad 331 may perform the foot bath function by conducting a temperature atmosphere controlled by an external force to the user's calf.

The side module 300 rotating forward may be when the inlet 510 is opened so that the user's leg is inserted into the action space 500. Alternatively, the side module 300 may be moved forward even when the user withdraws their leg from the action space 500. The side module 300 being moved backward may be when the inlet 510 decreases in size, and the action space is contracted, or the calf contacts the calf contact pad 331 after the user inserts their leg.

When the upper module 200 and the side module 300 open the inlet 510, the upper module 200 may start the opening thereof first, and then, the side module 300 may be opened. This is done because the upper module 200 performs the sliding operation, while the side module 300 rotates, and thus, if the side module 300 rotates forward first, the side module 300 may interfere with the upper module 200.

When the upper module 200 and the side module 300 close the inlet 510, the upper module 200 and the side module 300 may be operated in reverse. For example, the side module 300 may be closed first at a predetermined angle, and then the side module 300 may be closed. Since the respective modules are operated in this order, the interference between the modules may be prevented.

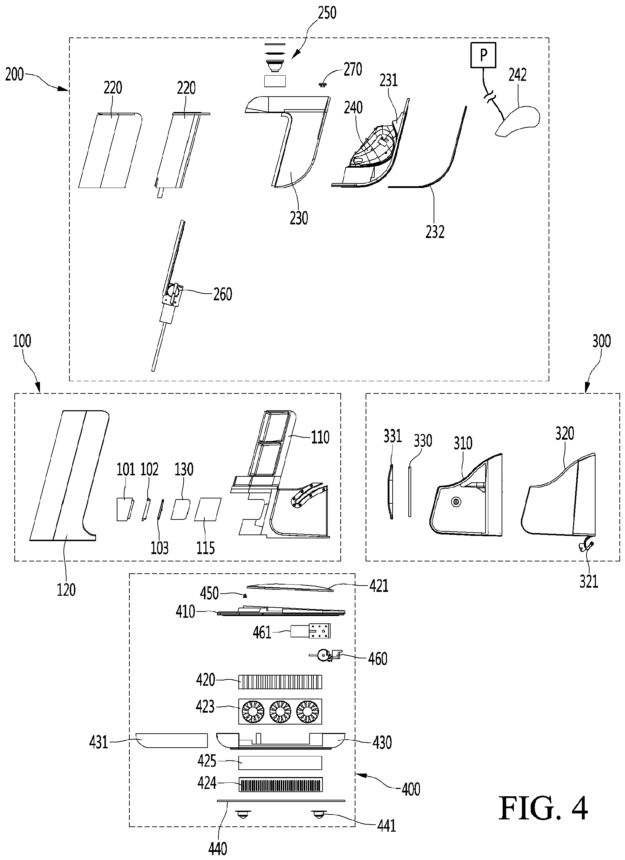

FIG. 4 is an exploded side view of the entire leg care apparatus according to an embodiment. Constituents of each module of the leg care apparatus according to the embodiment will be described with reference to FIG. 4.

First, the main body 100 is provided with a main frame 110 and a main body outer cover 120 provided on a rear surface of the main frame 110. A predetermined empty space may be provided between the main frame 110 and the main body outer cover 120, and components required for operating the leg care apparatus may be accommodated in the empty space.

The main body 100 may include a blower 101, a fragrance case 102 for accommodating a fragrance kit 103, and an atomizer 130. In addition, a heat generator, a radiant heater, and a cooler may be further provided.

The blower 101 is a device for generating a forced air current in the action space 500. The fragrance kit 103 may be provided as a device that provides fragrance into the action space 500 or remove a smell from the action space 500.

The atomizer 130 may supply mist to the inside of the action space 500 in at least one manner selected from ultrasonic spraying and heating spraying of water. A case in which both types of mist providing manners are installed may also be included in the embodiment.

The upper module 200 includes an upper frame 210 to which a portion of a movable member of the vertical opening device 260 is fixed to be elevated with respect to the main frame 100. An upper inner cover 230 and an upper outer cover 220 may be provided at an inner side and an outer side of the upper frame 210, respectively, to define an outer appearance of the upper module 200.

The knee care part 240, and a knee care seating panel 232 for mounting the knee care part 240 may be provided in front of the upper inner cover 230.

The side module 300 may include a side frame 310 and a side outer cover 320 provided in front of the side frame 310.

A calf thermoelectric module 330 and the calf contact pad 331 may be provided on an inner surface of the side module 300. A thermoelectric element may be provided in the calf thermoelectric module 330 to supply cold and hot air as desired by the user.

The bottom module 400 includes a bottom frame 410, a bottom housing 430 accommodating an outer edge of the bottom frame 410, and a bottom plate 440 that opens and closes a lower portion of the bottom frame 410.

A bottom supporter 441 provided as a wheel or the like is provided on a bottom surface of the bottom plate 440 so that the user may easily move the leg care apparatus.

The foot thermoelectric module 420 and the foot contact pad 421 that transfers the cold and hot air of the foot thermoelectric module 420 to the user's foot in a conduction manner may be provided inside the bottom housing 430. The foot thermoelectric module 420 and the foot contact pad 421 may contact each other to transfer heat. The heat exchange fan 423, the grill 424, and the filter 425 may be further provided as constituents for the hot or cold air that is exhausted from the foot thermoelectric module 420 to the outside.

The front and rear opening device 460 may be accommodated in the bottom housing 430 so that the side module 430 rotates. The front and rear opening device 460 may be provided with a rotation driving part 461 including at least a motor and a link driving part 462 including a power transmission part such as a gear.

The bottom housing 430 is provided with a light emitting element 450 that is exposed upward so that heat is irradiated to the user's foot. In this case, the light emitting element may irradiate infrared rays. The light emitting element 450 may be provided as an ultraviolet lamp to sterilize and disinfect the action space 500.

A water tray 431 that stores water to be discharged and through which the stored waste water is removed as necessary may be further provided at one side of the bottom housing 430. Water condensed after being atomized from the atomizer 130 to perform a predetermined function may be dropped into and stored in the water tray 431.

Hereinafter, each constituent of the leg care apparatus will be described in more detail.

FIG. 5 is an exploded side view of the main body. A configuration and operation of the main body will be described in more detail with reference to FIGS. 4 and 5.

Referring to FIGS. 4 and 5, the main body 100 may be largely divided into a main frame 110 that defines an overall shape of the leg care apparatus and supports a load of the leg care apparatus, and a main body outer cover 120 providing a predetermined space for accommodating components between the main body outer cover 120 and the main frame 110 and disposed behind the main frame 110.

At least a portion of the upper module 200 may be inserted into an interval between the main frame 110 and the main body outer cover 120, and thus, the upper module 200 may be vertically movable in a state of being guided to the main body 100. For this, the vertical opening device 260 may be accommodated in the interval between the main frame 110 and the main body outer cover 120.

The main frame 110 may be provided with a main front and rear extension part 112 extending forward and backward from a lower portion thereof and a main vertical extension part 111 extending upward from a rear portion of the main front and rear extension part 112. The main vertical extension part 111 may extend forward in a state of being inclined at a predetermined angle β with respect to the main front and rear extension part 112. The predetermined angle may be an acute angle. Thus, the user may insert their leg into the action space 500 in a more comfortable posture and use the leg care apparatus.

The main front and rear extension part 112 may be provided to close both sides of the lower portion of the action space 500. Thus, the forced air current within the action space 500 may not be lost through both side surfaces of the action space 500.

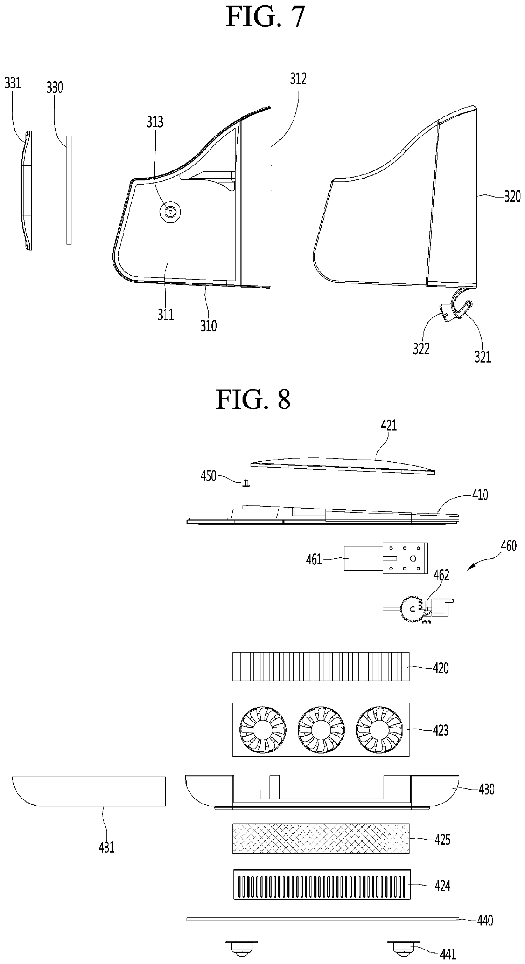

A guide slot 115 that guides the rotation of the side module 300 may be provided in the main front and rear extension part 112. The guide slot 115 may be provided to open the main front and rear extension part 112 in a curved shape and also be provided to define a groove having a curved shape in the main front and rear extension part 112. A protrusion (see reference numeral 313 of FIG. 7) of the side module 300 may be placed to be guided within the guide slot 115.

To guide the protrusion 313, the guide slot 115 may be provided as a curve having a geometric center with respect to a predetermined rotation center point C. The guide slot 115 may be provided in a curved shape having a predetermined length L as a curvature radius at the rotational center point C. The rotation center point C may be one point of a movement support part (see reference numeral 321 of FIG. 7) of the side module 300.

The main front and rear extension part 112 is completely closed except for a region of the guide slot 115. The guide slot 115 may be completely covered by the side frame 310 of the side module 300. This is the same as in a case in which the side module 330 completely rotates forward to be opened. Thus, both spaces of the action space 500 may be completely covered, and the forced air, which is artificially manipulated, in the action space 500 may leak to the outside.

For this, the side frame 310 may accommodate the main front and rear extension part 112 therein. Also, a flow blocking film 1121 that blocks the air leakage of the action space may extend up to an upper end of the main front and rear extension part 112. The flow blocking film 1121 may block the action space 500 even when the side module 300 is opened to cover the inside of the action space 500 from the outside.

An operation of the flow blocking film 1121 may be seen in FIG. 2. FIG. 2 illustrates a state in which the flow blocking film 1121 is exposed to the outside of a side portion side surface part 311 to cover the action space 500 in a state in which the side module 300 is opened.

Referring to FIG. 5, a main rear surface part 113 having a rear opening 114 may be provided on a rear surface of the main front and rear extension part 112. Components that provide various atmospheres required for the operation of the action space 500 may be mounted at a rear side of the main rear surface part 113. An operation medium that provides an atmosphere of the action space 500, such as air, light, and mist may pass through the rear surface opening 114.

The components that are placed at the rear side of the main rear surface part 113 may include the blower 101 that performs a blowing operation, the fragrance kit 103 that cleanly maintains the action space, the fragrance case 102 in which the fragrance kit 103 is accommodated, and the atomizer 130 that provides mist. Alternatively, other components may be further provided for a smooth operation of the action space 500.

The blower 101 may suction air from at least one of the inside or the outside of the action space 500 to supply the air to the action space 500. Here, the air supplied into the action space 500 may be artificially controlled in temperature. To control the temperature, the blower 101 may be provided with a separate temperature controller that is exemplified as the heat generator and the cooler.

The blower 101 may suction air within the action space 500 to apply a predetermined artificial operation to the suctioned air, thereby supplying the air into the action space 500. This may be understood as an air circulation inside the action space 500. Accordingly, energy efficiency may be improved by reducing the operation medium disposed to the outside.

An example of the fragrance kit 103 may include perfume and a photocatalyst smell decomposition device. The perfume may be a component that supplies an artificially good smell. The photocatalyst smell decomposition device is a member that is exemplified as titanium oxide and may be a device for decomposing smell particles by a catalytic action using action light such as ultraviolet light.

The atomizer 130 is a device for supplying mist. When the atomizer 130 is operated in the ultrasonic spraying manner, the mist may be supplied to the inside of the action space 500 without being hot, the legs may be cared while being cool, and the inside of the action space 500 may be cool through latent heat and the like. When the atomizer 130 is operated in the heating spray manner, the mist may be supplied to the inside of the action space 500 in a hot state, the leg may be warmed while taking the foot bath, and the inside of the action space 500 may be warmed.

The atomizer 130 may be provided with an ultrasonic spray device and a heating spray device. In this case, since the leg care apparatus is used in more various manners, the user's satisfaction may be improved.

The mist supplied from the atomizer 130 may perform a predetermined action in the action space 500.

For example, the high-temperature mist contacting the user's leg may transfer heat to the user's leg in a conduction manner. The high-temperature mist may be condensed on a surface of the user's leg and then heated by external hot air so that the foot bath is performed by continuously transferring heat to the user's leg in the conduction manner. For another example, the mist condensed on the user's leg may be evaporated to take the cold fomentation on the user's leg.

The rear surface opening 114 may be closed by the main rear cover 116. The main rear cover 116 may be provided in a shape in which a hole is processed to allow the operation medium to pass therethrough.

The main body outer cover 120 may be provided in a shape that is inclined forward toward an upper side, like the main vertical extension part 111.

FIG. 6 is an exploded side view of the upper module 200. A configuration and operation of the upper module 200 will be described in more detail with reference to FIGS. 4 and 6.

Referring to FIGS. 4 and 6, the upper module 200 may be moved upward or downward with respect to the main body 100 by the vertical opening device 260. Here, the upper frame 210 may be a component constituting a frame of the upper module 200 and extend to be obliquely inclined forward like the main frame 110.

The vertical opening device 260 may include an elevation driving part 261 including at least a motor, an upper rail 262 extending upward from the elevation driving part 261, and a lower rail 263 extending downward from the elevation driving part 261. The upper rail 262 may be coupled directly or indirectly to the upper frame 210. The lower rail 263 may be coupled directly or indirectly to the main frame 110. At least one of the upper rail 262 or the lower rail 263 may be moved to allow the upper module 200 to ascend or descend.

The upper inner cover 230 and the upper outer cover 220 may be respectively coupled to front and rear portions of the upper frame 210 to define an outer appearance of the leg care apparatus. When the upper frame 210 is moved, the upper inner cover 230 and the upper outer cover 220 may be moved together.

The upper inner cover 230 may include an upper side surface part 233 extending vertically and inclined forward and an upper portion top surface part 234 extending backward from an upper end of the upper side surface part 233 and providing an upper end surface of the leg care apparatus.

The upper portion top surface part 234 may be a surface that is mainly observed when the user uses the leg care apparatus, and thus may be used variously. For example, the upper portion top surface part 234 may be provided with a water supply device 250 that supplies water used in the atomizer 130 and a display 270 that allows the user to control the leg care apparatus.

The water supply device 250 may include a water supply frame 254 in which the supplied water is primarily stored, a water supply supporter 253 that injects water into the water supply frame 254, and a water supply seating panel 252 that supports a water supply cover 251. The user may conveniently supply water by using the water supply device 250.

The display 270 may display information that is necessary for the operation of the leg care apparatus. Manipulation information that is necessary for controlling the leg care apparatus may be inputted by using the display 270. The display 270 may be provided as a touch panel.

The knee care part 240 may be disposed on a front portion of the upper module 200 to care the user's knee. The knee care part 240 may be provided to the knee care frame 231. To allow the knee care frame 231 to be coupled to the upper inner cover 230, a knee care seating panel 232 may be further provided.

The knee care part 240 may include at least one light emitting element 241 that irradiates infrared rays to the knee, at least one massage pad 242 that presses a spaced peripheral portion of patella, and a pump P that controls an air pressure to the inside of the massage pad 242. The massage pad 242 may be applied in other methods such as spring pressure control rather than the air pressure control.

The knee care part 240 may include a knee placing part 243. The at least one light emitting element 241 and the at least one massage pad 242 are placed at positions of an inner region of the knee placing part 243, respectively. The knee placing part 243 may be a structure in which a material such as a soft cushion is filled and may apply an overall pressure to the user's knee to care the knee comfortably. According to the knee placing part 243, the action due to the massage pad 242 may be more improved.

Unlike the action space 500, as described above, the knee care part 240 performs an action such as pain relief of the knee by applying pressure and heat.

FIG. 7 is an exploded side view of the side module 300. A configuration and operation of the side module 300 will be described in more detail with reference to FIGS. 4 and 7.

Referring to FIGS. 4 and 7, the side module 300 rotates backward and forward so that the user may conveniently use the leg care apparatus.

The side module 300 may include a side frame 310 connected to the main frame 110 and a side outer cover 320 provided in front of the side frame 310.

The side frame 310 may include a side portion front surface part 312 and a side portion side surface part 311 extending backward from both sides of the side portion front surface part 312. The side portion side surface part 311 may be provided as two left and right walls, and the main front and rear extension part 112 may be inserted into an inner spaces of the two walls.

A protrusion 313 may be provided inside the side portion side surface part 311, and the protrusion 313 may be guided by the guide slot 115 (see FIG. 5). The positions at which the protrusion 313 and the guide slot 115 are provided may be opposite to each other. However, for stable operation, it is preferable that the protrusion 313 is provided on the side module 300, and the guide slot 115 is provided on the main body 100.

A movement support part 321 supporting the rotation operation of the side module 300 may be provided on a lower portion of a front end of the side outer cover 320. The movement support part 321 may be hung and supported at any point of the bottom module 400 or the main body 100. The movement support part 321 may act as a center point of relative rotation with respect to the main body 100 of the side module 300.

A movement contact part 322 is provided at an adjacent position of the movement support part 321 to receive driving force of the link driving part 462 (see Fig. 8). For example, the link driving part 462 and the movement contact part 322 may be engaged with each other to receive the driving force of the rotation driving part 461.

An interaction between the main body 100 and the side module 300 may be performed by the rotation operation through the transmission of the driving force of the front and rear opening device 460 and the guiding action of the protrusion 313 and the guide slot 115.

The rotation driving force may be transmitted from the bottom module 400 to the side module 300 by the action connected to the rotation driving part 461, the link driving part 462, and the movement contact part 322 in time series. Here, the side module 300 may rotate in a state of being supported by the movement support part 321.

When the side module 300 is rotated by the rotation driving force, the protrusion 313 of the side module 300 may be guided by being placed inside the guide slot 115. The side module 300 may rotate at a curvature radius by a correct rotation center by the mutual guiding action of the guide slot 115 and the protrusion 313.

A calf thermoelectric module 330 and the calf contact pad 331 may be provided on an inner surface of the side module 300. A thermoelectric element may be provided in the calf thermoelectric module 330 to supply cold and hot air as desired by the user. Accordingly, the foot bath function for the calf portion of the user may be performed.

When the calf thermoelectric module 330 has a large heat load, a separate heat exchange fan may be installed like the foot thermoelectric module 420.

FIG. 8 is an exploded side view of the bottom module 400. A configuration and operation of the bottom module 400 will be described in more detail with reference to FIGS. 4 and 8.

Referring to FIGS. 4 and 8, a plurality of components for the foot bath may be provided in the bottom module 400. The bottom module 400 includes a bottom frame 410, a bottom housing 430 accommodating an outer edge of the bottom frame 410, and a bottom plate 440 that opens and closes a lower portion of the bottom frame 410.

The foot thermoelectric module 420 and the foot contact pad 421 that transfers the cold and hot air of the foot thermoelectric module 420 to the user's foot in a conduction manner may be provided inside the bottom housing 430. The foot thermoelectric module 420 and the foot contact pad 421 may contact each other to transfer heat. The foot contact pad 421 may contact the sole of the user, and the hot or cold air may be transferred to the sole of the foot to perform the foot bath function.

The foot contact pad 421 may be made of a metal having high thermal conductivity, for example, copper or stainless steel so as to uniformly transfer heat to the entire sole of the foot. This may be equally applied to the calf contact pad 331.

When water having a predetermined level is accumulated in the bottom housing 430, the foot contact pad 421 may heat the accumulated water to perform the foot bath function for the foot.

The heat exchange fan 423, the grill 424, and the filter 425 may be further provided as constituents for the hot or cold air that is exhausted from the foot thermoelectric module 420 to the outside. High energy may be supplied to the foot thermoelectric module 420 to supply a large amount of hot or cold air when compared to the calf thermoelectric module 330. Heat generated in and exhausted from the thermoelectric module 420 may be smoothly discharged to the outside by the heat exchange fan 423.

To allow the air circulated to the heat exchange fan 423 to perform a cooling operation without any problem, the grill 424 and the filter 425 may be provided. The air in which foreign substances are filtered by the filter 425 may be supplied to the blower 101 and supplied to the action space 500. In this case, cleaner air may be supplied to the action space 500 to improve the user's satisfaction.

The front and rear opening devices 460 are accommodated in the bottom housing 430 to allow the side module 430 to rotate as described above. A large portion of the front and rear opening device 460 is accommodated in the bottom module 400, but is not limited thereto. For example, the front and rear opening device 460 may be provided to the main body 100.

The bottom housing 430 is provided with a light emitting element 450 that is exposed upward so that heat is irradiated to the user's foot. The light emitting element 450 may perform various functions such as sterilization, ultraviolet light for photocatalytic decomposition, infrared rays, and the like depending on the emitted light.

The water tray 431 that stores waste water to be discharged and wastes may be further provided at one side of the bottom housing 430. In the water tray 431, water condensed after being atomized by the atomizer 130 may be dropped into and stored. The water tray 431 is provided as a component that is slid to be separated to the outside. A valve may be provided in a passage through which water flows into the water tray 431 to prevent the water from leaking during the foot bath.

A bottom supporter 441 provided as a wheel or the like is provided on a bottom surface of the bottom plate 440 so that the user may easily move the leg care apparatus. The bottom supporter 441 is provided as a rotatable wheel so that the user conveniently moves and uses the leg care apparatus in various directions. In the case of the elderly, the advantages of the above-described moving device may be largely utilized.

The leg care apparatus according to an embodiment is provided with an atomizer to spray the mist into the inside of the action space 500. The mist may cover the user's leg in the action space 500.

The mist that covers the user's leg may be heated by heat transmitted from the outside and may transfer the heat to the user's leg while the user is taking the foot bath. In other cases, the mist that covers the user's leg may be cooled by cold air transferred from the outside or may be evaporated to absorb heat to take the cold fomentation by the absorbed heat.

Hereinafter, a configuration of the atomizer will be described in detail. In the following description, the atomizer and constituents related to the atomizer may be emphasized for ease of understanding.

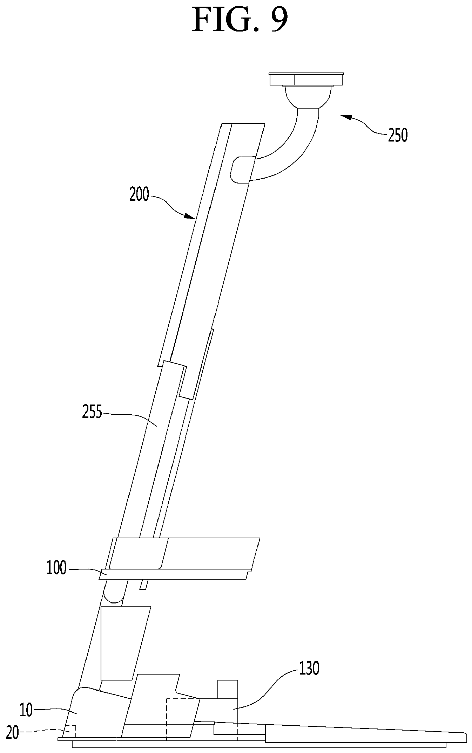

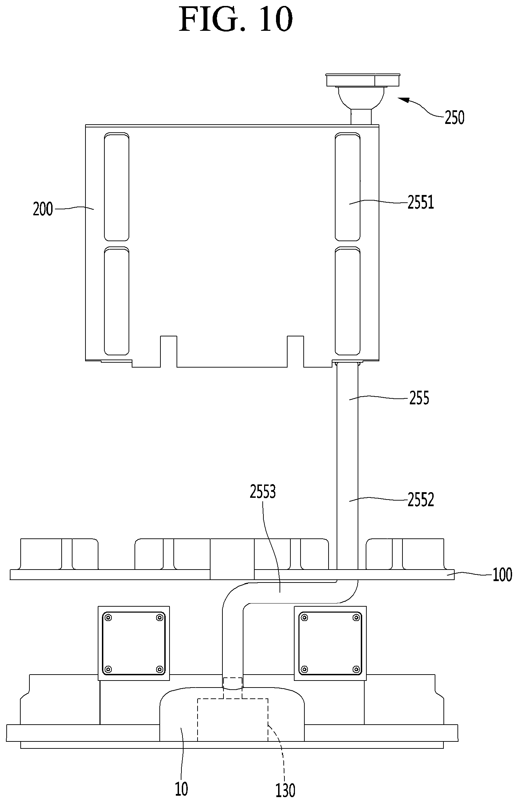

FIG. 9 is a side view of the leg care apparatus showing constituents related to the atomizer, and FIG. 10 is a rear view of the leg care apparatus showing the constituents related to the atomizer.

Referring to FIGS. 9 and 10, the leg care apparatus according to the embodiment may include an atomizer 130, a water tank 10 that supplies water to the atomizer 130, a water supply device 250 that supplies water to the water tank 10, and a conduct pipe 255 which connects the water supply device 250 to the water tank 10 and through which water passes.

The atomizer 130 is a device that provides mist by applying ultrasonic waves to water. The water tank 10 is configured to secure functional stability of the atomizer 130 by supplying a predetermined amount of water to the atomizer 130. The water supply device 250 is a device in which the user supplies water from the outside. The water supply device 250 is partially exposed through the top surface of the upper module 200 so that the user may conveniently supply water.

The water supply device 250 may be disposed at one side edge of the upper module 200. When the user uses an upper portion top surface part 234 (see FIG. 6) of the upper module 200 as a table, the upper portion top surface part 234 partially exposing the water supply device 250 should not interfere with the user. For example, when the user places an object such as a book on the upper portion top surface part 234, the water supply device 250 may be disposed at one corner of the upper portion top surface part 234 so as not to interfere with the book.

The water supply device 250 may be provided in the upper module 200, and the upper module 200 may be moved vertically with respect to the main body 100. The water supply device 250 is provided at one corner of the upper portion top surface part 234.

To allow the conduct pipe 255 to connect the water supply device 250 to the water storage tank 10, the conduct pipe 255 may be provided in a pipe structure including two pipes that are mutually expandable. For example, the conduct pipe 255 may include a first conduct pipe 2551 connected to one side of the water supply device 250 and a second conduct pipe 2552 connected to one side of the water tank 10.

The other sides of the first conduct pipe 2551 and the second conduct pipe 2552 may overlap with each other by a predetermined length. Even if the first conduct pipe 2551 and the second conduct pipe 2552 are spaced apart from each other in a longitudinal direction, the pipe connection of the first conduct pipe 2551 and the second conduct pipe 2552 may not be disconnected. Even if the upper module 200 is moved upward from the main body 100, the conduct pipe 255 may guide water from the water supply device 250 to the water tank 10 without water leakage.

The conduct pipe 255 may be provided in a form of a corrugated pipe having a stretchable length when not in a configuration in which two pipes overlap with each other. As another configuration, the conduct pipe 225 may be provided as a corrugated pipe having a predetermined length so as to be stretchable, and also be provided as a rigid pipe for coupling by a predetermined length.

At least a portion of each of the first conduct pipe 2551 and the second conduct pipe 2552 may be bent. In the embodiment, the second conduct pipe 2552 may be bent towards a center to provide a conduct pipe bending part 2553. The conduct pipe bending part 2553 is configured to guide water towards the water tank 10 even if the water supply device 250 is provided at one corner of the upper portion top surface part 234. The water tank 10 may be disposed at the center of the main body 100 with respect to the horizontal direction.

The atomizer 130 may be provided in front of the water tank 10. The atomizer 130 may be adjacent to the action space 500 as closely as possible so that the generated mist is directly supplied to the action space 500 without condensation.

The atomizer 130 may be operated using ultrasonic waves. The mist that is in a room-temperature state may be provided using a phenomenon in which water molecules are evaporated from the surface by vibration of the ultrasonic waves.

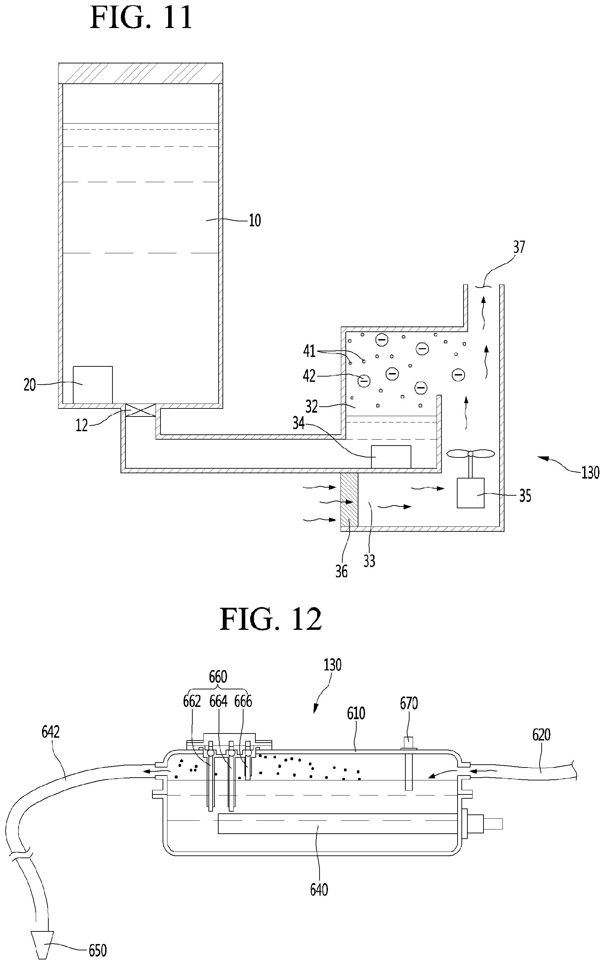

FIG. 11 is a schematic view of the atomizer.

The atomizer 130 may be supplied with water from the water tank 10 to generate fine water particles 41 and anion 42 so as to be sprayed to the outside.

A mist generator 32 having one end coupled to the lower portion of the water tank 10 and the other end coupled to the atomizer 130 is provided. A flow rate control valve 12 is provided at the coupling portion of the water tank 10 and the mist generator 32 to maintain a water level of the mist generator 32 at a predetermined level.

A vibrator 34 may be installed on a bottom surface of the mist generator 32. The vibrator 34 may vibrate the water contained in the mist generator 32 to generate fine water particles 41. Here, a frequency of the vibrator 34 belongs to a region of the ultrasonic wave that is inaudible to the human ear.

A suction hole 33 is defined in one side of the atomizer 130. A spray fan 35 that suctions external air through the suction hole 33 is installed at one side of the mist generator 32. A filter 36 that filters foreign substances contained in the suctioned air may be installed on the suction hole 33. In an embodiment, a prefilter may be installed as a filter for filtering the suctioned air.

It is preferable that a catechin component or an antibacterial component of green tea is applied to the filter 36 to kill bacteria suctioned together with the suctioned air into the filter 36.

A nozzle 37 through which the fine water particles 41 are sprayed to the outside may be installed above the atomizer 130. The nozzle 37 is configured to spray the mist into the action space 500 by loading the mist such as the fine water particles 41 and the anion 42 in the air current blown from the spray fan 35.

A sterilizer that is capable of sterilizing bacteria inhabited in the water stored in the water tank 10 may be provided in the water tank 10. An ultrasonic cell crusher 20 may be installed as the sterilizer.

The mist supplied from the nozzle 37 may be loaded in the strong air current provided by the separate blower 101 to reach every corner of the inside of the action space 500. In addition, the mist supplied from the nozzle 37 may be concentrately supplied to a portion that is adjacent to the nozzle 37 by being loaded in the weak air current provided by the spray fan 35.

The use of the blower 101 may be selected according to various modes of the user's preference and the foot bath. To allow the mist to reach the entire area of the user's leg placed in the action space 500, the blower 101 may be operated.

The foot bath function by the atomizer 130 will be described in more detail.

The mist supplied by the atomizer 130 may cover the user's leg in the action space 500. Thereafter, when the blower 101 provides an air current, the air current may be generated in the action space 500, and the mist that covers the user's leg may be evaporated. The mist may absorb heat from the leg while being evaporated to cool the leg. Thus, the user may enjoy a feeling of being cool.

The cold air of the air current within the action space 500 may be supplied to the user by providing a separate refrigeration cycle to the blower 101 or by providing a thermoelectric module exposed to the blower 101 or the action space 500. In this case, the cool feeling enjoyed by the user may increase even more. The thermoelectric module may include the foot thermoelectric module 420 and/or the calf thermoelectric module 330.

When the blower device 101 provides a hot air current, the air current in the action space 500 may heat the mist that covers the user's leg. The heated mist may heat the legs so that the user enjoys a hot foot bath.

The hot air current of the blower 101 may be achieved by the blower having a separate heater. As another method, the air current within the action space may form a hot atmosphere through a method in which the thermoelectric module is exposed to the action space or the separate heater is provided. The thermoelectric module may include the foot thermoelectric module 420 and/or the calf thermoelectric module 330.

Hereinafter, an embodiment of a high-temperature atomizer that provides high-temperature mist will be described. A related system of the leg care apparatus including the water tank 10 is the same as a room-temperature atomizer of FIGS. 9 to 11. Since the atomizer according to a following embodiment provides high-temperature mist unlike the room-temperature atomizer, the atomizer may be called a high-temperature atomizer.

FIG. 12 is a cross-sectional view illustrating an example of the atomizer. Referring to FIG. 12, the atomizer 130 will be described in detail as follows.

Referring to FIG. 12, the atomizer 130 may include a tank 610 in which water is stored, a heater 640 mounted in the tank 610, a water level sensor 660 that measures a water level of the atomizer 130, and a temperature sensor 670 that measures a temperature of the atomizer 130.

The water level sensor 660 may be constituted by a common electrode 662, a low water level electrode 664, and a high water level electrode 666. A high water level and a low water level may be sensed by whether the common electrode 662 and the high water level electrode 666 are electrically connected to each other or whether the common electrode 662 and the low water level sensor 664 are electrically connected to each other.

A water supply hose 620 that supplies water may be connected to one side of the atomizer 130, and a steam supply line 642 that discharges steam in one form of mist may be connected to the other side of the atomizer 130. A nozzle 650 having a predetermined shape may be provided at a front end of the steam supply line 642.

The water supply hose 620 may have one end connected to a water supply part that supplies water to the atomizer, and the nozzle 650 disposed at the front end of the steam supply line 642, i.e., a steam discharge hole may be connected to a predetermined position on the inner surface of the action space 500 to spray the high-temperature mist into the action space 500.

Here, the water supply part may be connected to the water tank 10. A valve may be provided at a connection portion with the water tank 10.

Although the atomizer 130 (hereinafter, for convenience of description, referred to as a "tank heating type") generates mist in a manner in which a predetermined amount of water stored in the tank 610 having a predetermined size is heated by the heater 640 is illustrated and described in the embodiment, the embodiment of the present disclosure is not limited thereto.

That is, in this embodiment, any device capable of generating high-temperature mist may be used as the atomizer. For example, a heater may be installed directly around or inside a predetermined case through which water passes. Accordingly, the water may be heated without being stored in a predetermined tank 610 (hereinafter, for convenience of description, referred to as a "pipe heating type"). In the pipe heating type atomizer, water introduced to flow into the atomizer may be heated and converted into high-temperature mist.

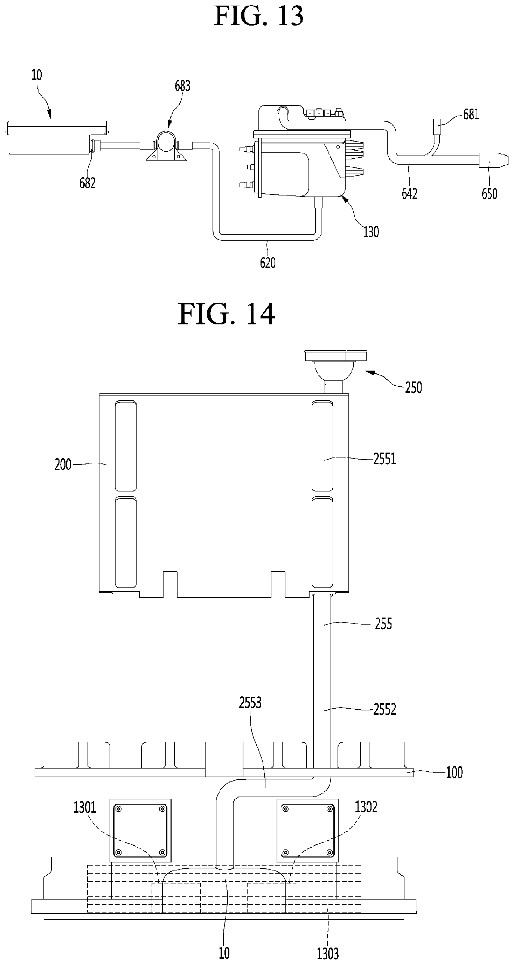

FIG. 13 is a schematic view of a system related to the atomizer.

Referring to FIG. 13, in this embodiment, the water tank 10 may store a predetermined amount of water as a water supply part that supplies water to the atomizer 130 and may be provided behind the atomizer 130.

A pump 683 may be provided between the water tank 10 and the atomizer 130. The pump 683 may be rotatable forward and backward to supply water to the atomizer 130 or to collect remaining water in the atomizer 130 as necessary. The collection of the residual water as described above is intended to prevent or minimize the accumulation of inorganic matters within the atomizer such as scale. For example, if an amount of inorganic matters contained in the water is small or negligible, a forward-only pump may be used.

The residual water collected into the water tank 10 may be discharged by emptying the water tank 10. Here, the concentration of the inorganic material may be high.

Since the pump 683 that is rotatable forward and backward is used, it may be possible to prevent the failure of the atomizer 130. Specifically, in the high-temperature atomizer used in a manner in which water is boiled to provide mist, after the water is boiled, the inorganic matters remain in the tank 610. The remaining inorganic matters may cause the failure of the atomizer 130. To prevent this failure, when the atomizer is operated for a predetermined time, and the residual water remains by a predetermined level or less, the inorganic matters may be reduced by removing the residual water in the tank 610.

The pump may be replaced with an on/off valve. This may be possible by using a water level difference between the water tank 10 and the atomizer 130. That is, it may be possible to supply water from the water tank 10 to the atomizer 130 by using gravity. In this case, the on/off valve may be turned on to automatically supply the water from the water supply part to the atomizer.

The water may be supplied to a lower portion of the atomizer 130, and the steam may be discharged from an upper portion of the atomizer 130. This may be advantageous for collecting the residual water of the atomizer 130. Of course, as illustrated in FIG. 12, the water may be supplied to an upper portion of the atomizer 130. In this case, a separate drain structure for collecting the residual water may be provided.

A safety valve 681 may be provided in a steam passage that discharges steam from the atomizer 130, i.e., the steam supply line 642. This may be done for preventing a safety accident by preventing a steam pressure from increasing when the steam passage, in particular, the nozzle 650 is blocked.

The foot bath function according to the high-temperature atomizer will be described in more detail.

The high-temperature mist supplied by the atomizer 130 may directly cover the leg of the user, or the high-temperature mist may condense on the leg to perform the foot bath function.

The blower 101 may be additionally operate to allow the high-temperature mist to reach the entire leg area of the user.

When the blower 101 provides a hot air current, the air current in the action space 500 may continuously heat the mist that covers the user's leg. In this case, even when the hot mist is cooled, the foot bath may be continuously performed.

The hot air current of the blower 101 may be achieved by the blower 101 having a separate heater. As another method, the air current within the action space 500 may form a hot atmosphere through a method in which the thermoelectric module exposed to the action space or the separate heater is provided. The thermoelectric module may include the foot thermoelectric module 420 and/or the calf thermoelectric module 330.

The atomizer of FIGS. 9 to 11 may be a room-temperature atomizer that is operated by ultrasonic waves, and the atomizer of FIGS. 12 and 13 may be a high-temperature atomizer that is operated in a heating manner.

As described above, the room-temperature atomizer or the high-temperature atomizer may be provided separately in the leg care apparatus. The room-temperature atomizer and the high-temperature atomizer may be provided together in the leg care apparatus. Thus, the user may enjoy the foot bath in various ways regardless of a temperature, such as cold or hot fomentation.

FIG. 14 is a schematic rear view of the leg care apparatus in which the room-temperature atomizer and the high-temperature atomizer are installed together. Since other parts are the same in FIG. 14, the already described contents are applied herein, and the description thereof is omitted, and thus, only the parts related to the atomization apparatus will be described.

Referring to FIG. 14, the room-temperature atomizer 1301 capable of providing mist at room temperature and the high-temperature atomizer 1302 capable of providing high-temperature mist may be provided on both sides of the water tank 10.

The mist provided by the room-temperature atomizer 1301 and the high-temperature atomizer 1302 may be widely supplied into the action space 500 so that the mist is evenly distributed on the user's leg surface. For this, a mist supply slit 1303 having a slit structure that is provided lengthily in a left and right direction at the lower portion of the main vertical extension part 111 (see FIG. 5) may be provided.

Since the mist supply slit 1303 is provided lengthily in the left and right direction, the steam may be supplied to both left and right legs of the user. Since the mist supply slit 1303 is provided lengthily, the mist may be prevented from being concentrated and supplied to one portion of the leg. A diffuser may be provided between the nozzles 37 and 650 and the mist supply slit 1303.

Since a discharge hole of the mist supply slit 1303 is provided widely, the mist may be not concentrated to one portion of the leg, and burns due to the high-temperature mist may be prevented. For this, the diffuser and a baffle may be provided between the nozzle 650 and the mist supply slit 1303.

To smoothly supply the mist into the action space 500, the blower 101 may be operated together when the mist is supplied.

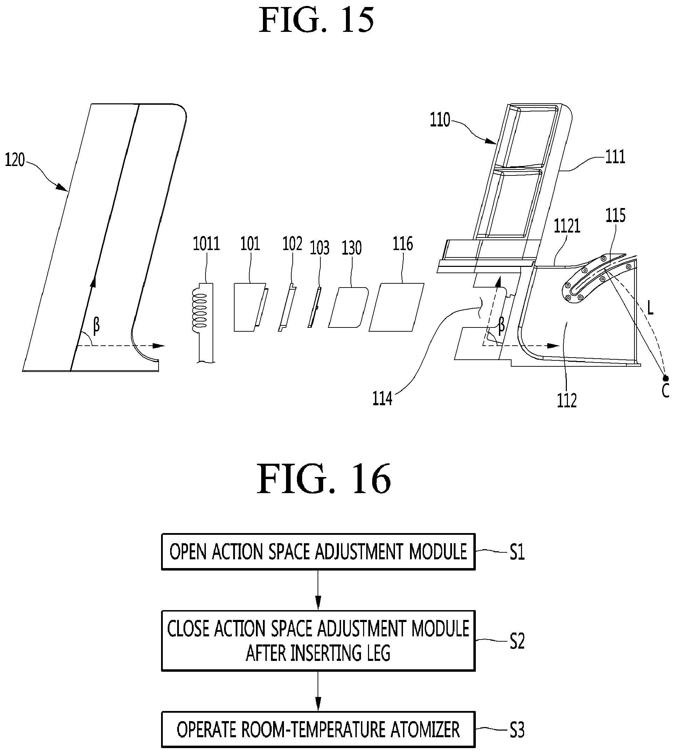

FIG. 15 is an exploded side view illustrating a main body of a leg care apparatus according to another embodiment. Other parts of the leg care apparatus, which are not illustrated in FIG. 5 may be applied to the description of the foregoing embodiment as it is. The description of the original embodiment will be applied to the parts, which are not described, as it is.

Referring to FIG. 15, according to another embodiment, a heating wire 1011 that provides heat to an air current provided by a blower 101 may be further provided. The heating wire 1011 may provide hot air to the action space 500 by loading hot air in the air current when the blower 101 is operated to provide the air current with hot air into the action space 500.

The high-temperature air of the air current may heat the user's legs or the mist inside the action space 500.

The leg care apparatus according to an embodiment may perform the foot bath in a cold atmosphere. The foot bath in the cold atmosphere may be referred to as cold fomentation. A method for controlling the leg care apparatus that performs the cold fomentation by using the leg care apparatus will be described below. The cold fomentation in the cold atmosphere may be utilized in hot summer.

FIG. 16 is a flowchart for explaining a method for controlling the leg care apparatus according to an embodiment. The method may be performed by a controller that may be a microprocessor, an integrated circuit, an electrical logical circuit, and the like. Subsequent flowcharts for explaining a method for controlling may also be performed by a controller.

Referring to FIG. 16, the user who wants to perform the cold fomentation opens the action space adjustment module (S1). The action space adjustment module may be sufficiently opened so that the user may insert their leg.

After the user inserts their leg through the inlet 510, the action space adjustment module is closed (S2). Here, a size of the action space may be adjusted to fit the user's leg.

Thereafter, the room-temperature atomizer 1301 is operated (S3). As described above, the room-temperature atomizer 1301 may blow fine water particles at room temperature by using ultrasonic waves. The fine water particles sprayed in the room-temperature atomizer may be supplied into the action space 500 by a spray fan 35.

The fine moisture particles may cover the user's leg within the action space. Since the fine water particles have room temperature less than the body temperature, the fine water particles may take the cold fomentation by the fine water particles covering the user's legs.

The fine water particles may generate water droplets on the skin of the user's leg. The water droplets may be evaporated by body temperature and may absorb ambient heat. The leg may be cooled by the absorption heat. The cold fomentation taken by the user may be stronger.

In the method for controlling the leg care apparatus according to this embodiment, the cold fomentation may be performed on the leg in which the blood collects in the hot summer. Accordingly, the user may lead a more comfortable life.

According to the method for controlling the leg care apparatus described above, the user may enjoy the cold fomentation. The cold fomentation may be performed by two mechanisms such as heat conduction by the fine water particles at room temperature and heat absorption from the skin at the time of evaporation of water.

According to the two mechanisms, the cold fomentation is performed slowly, and thus, the temperature may not be significantly lowered during the cold fomentation. This is done because an amount of fine water particles supplied from the room-temperature atomizer 1301 is less than a predetermined amount, and an amount of water evaporated from the user's skin is also limited. In addition, there may be a user who wants to perform the cold fomentation at a lower temperature than the ambient temperature.

The following suggests a method that is capable of improving the above limitations to perform strong cold fomentation.

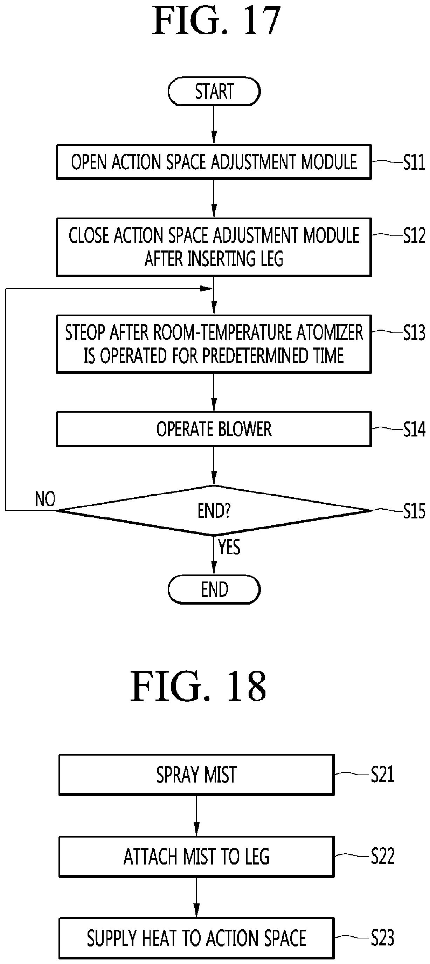

FIG. 17 is a flowchart for explaining a method for controlling a leg care apparatus according to another embodiment.

Referring to FIG. 17, opening an action space adjustment module (S11) and closing the action space adjustment module after inserting the legs (S12) are the same as the above-described embodiment.

Thereafter, mist is sprayed into the action space for a predetermined time by using the room-temperature atomizer 1301 and then stopped (S13). In this case, a spray fan 35 may be used for spraying the mist. After the spraying of the mist is stopped, a strong air current is blown into the action space by using the blower 101 (S14).

The strong airflow of the blower may quickly cool the skin by evaporating water covering the user's skin by forced convection. Accordingly, the user may have stronger cold feeling, and the strong cold fomentation may be performed.

Subsequently, it is determined whether the cold fomentation is ended (S15), and when continuing the cold fomentation, a temporary operation (S13) of the room-temperature atomizer and the operation (S14) of a blower may be repeatedly performed. The temporary operation (S13) of the room-temperature atomizer and the operation (S14) of the blower are repeated because the water adhering to the skin of the user is evaporated and lost.

The room-temperature atomizer and the blower may be operated together. However, in this case, the mist supplied from the room-temperature atomizer is not preferable because the mist is discharged to the outside together with the air current. In this case, humidity of an indoor space increases excessively due to the discharged mist, which is not preferable. However, in the method for controlling the leg care apparatus according to an embodiment, the room-temperature atomizer and the blower are not excluded from being operated together.

To perform the stronger cold fomentation, a calf thermoelectric module 330 and a thermoelectric module 420 may be operated. Here, as opposed to the case of the hot fomentation, a surface contacting the user's skin may achieve a cold atmosphere, and a surface that does not contact the skin may achieve a hot atmosphere.

An operation of the thermoelectric modules 330 and 420 may be performed from a time at which the user selects a function of the cold fomentation, i.e., from beginning of a cold fomentation mode. Accordingly, the cold feeling felt by the user may be enhanced more and faster.

Alternatively, the operation of the thermoelectric modules 330 and 420 may be performed together with the operation of the room-temperature atomizer 1301. Accordingly, the thermoelectric modules 330 and 420 may transfer cold air to the skin of the user indirectly through the fine water particles.

As a result, the user may be prevented from being surprised or having frostbite due to the sudden low temperature atmosphere. Since the water intervenes between the skin of the leg and contact pads 331 and 421, the entire area of the skin contacting the contact pad may be cooled.

The cold fomentation illustrated in FIG. 16 may be referred to as general cold fomentation because it is cold in a mild atmosphere, and the cold fomentation illustrated in FIG. 17 may be referred to as strong cold fomentation because it is cold and powerful quickly.

The leg care apparatus according to this embodiment may provide a foot bath atmosphere at a hot atmosphere. A method for controlling the leg care apparatus that is controlled to perform the foot bath according to the embodiment will be described below.

FIG. 18 is a flowchart for explaining a method for controlling a leg care apparatus according to another embodiment.

Referring to FIG. 18, first, mist is provided into the action space (S21). To provide the mist, at least one of a room-temperature atomizer 1301 or a high-temperature atomizer 1302 may be operated.

Various kinds of mist may be provided according to operations of the room-temperature atomizer 1301, the high-temperature atomizer 1302, a heating wire 1011, and a blower 101.

Specifically, when only the room-temperature atomizer 1301 is operated, room-temperature mist may be provided to an action space 500. When only the high-temperature atomizer 1302 is operated, high-temperature mist may be provided to the action space. When the room-temperature atomizer 1301 and the high-temperature atomizer 1302 are operated together, a large amount of mist may be provided to the action space at a temperature between room temperature and high temperature.

The operation of the room-temperature atomizer 1301 and the high-temperature atomizer 1302 may be selectively performed in response to a temperature atmosphere and a humidity atmosphere, which are desired by a user. For example, when a large amount of mist is intended to be supplied, the room-temperature atomizer 1301 and the high-temperature atomizer 1302 may be operated together even though the temperature is low. In another example, a case in which only the room-temperature atomizer 1301 is operated may be a case in which the user intends to perform a mist foot bath at room temperature at an operation starting time point.

The mist supplied to the action space 500 may cover the leg of the user to allow the user to enjoy the foot bath (S22) .

The mist covering the user's legs may be grown to form water droplets through an aggregation process and a condensation process. As the hot mist newly supplied to the aggregated droplets is aggregated, the aggregated droplets may be maintained at a predetermined temperature.

The aggregated water droplets may fall along the skin of the leg, and droplets may be newly formed by the newly supplied mist so that the user enjoys the foot bath. However, when a large amount of water is accommodated in the action space 500, even if a high-temperature mist is supplied, the amount of heat transferred to the legs may be small. For example, since a heat load inside the action space 500 increases as an amount of water in the action space 500 increases, a temperature of the action space 500 gradually decreases even when the same amount of mist is supplied.

As a result, the temperature inside the action space 500 decreases even if the mist is continuously supplied in the same state. Accordingly, the effects of the foot bath may be deteriorated.

In consideration of a large amount of water accommodated in the action space, heat may be supplied to the inside of the action space through other parts other than the mist (S23).

A method for supplying heat into the action space 500 may include, first, operating the blower 101 and the heating wire 1011 to provide a high-temperature air current. Second, operating at least one of the blower 101 or at least one of the thermoelectric modules 330 and 420 so that the heat generated in the thermoelectric module is supplied to the inside of the action space 500 by being loaded in the air current by the blower. Third, operating at least one of the blower 101, the heating wire 1011, or the thermoelectric modules 330 and 420 so that an appropriate amount of heat is transferred into the action space 500 Fourth, operating all of the blower 101, the heating wire 1011, and the thermoelectric module 330 and 420 so that the greatest amount of heat is transferred into the action space 500.

The heat supplied in the heat supply process (S23) described above may sufficiently heat the mist covering the leg so that the user continues to have the foot bath. Alternatively, the supplied heat may directly convection-heat the skin to perform the foot bath. This may be distinguished from the mist that conduction-heats the skin, and thus, the user may enjoy the foot bath by using the conductive heating and convection heating together.

According to a preferred embodiment, when the user intends to take the foot bath at the same temperature while the mist is continuously supplied, the method for supplying the heat may be gradually changed from the method in which relatively low heat is supplied to the method in which relatively high heat is supplied among the above-described four methods. Here, by comparing an amount of mist supplied with the heat supplied in the heat supply process (S23), any method may be selected and operated.

Alternatively, if desired by the user, the user may enjoy the foot bath by selecting the method for supplying the high-temperature heat.

When the mist is heated by other heat supply part other than the mist, the user may continue to enjoy the foot bath by the mist even if there is much moisture in the action space.

According to the method for controlling the leg care apparatus according to an embodiment, the user may perform the foot bath at the same temperature atmosphere regardless of an amount of the mist being continuously supplied.

In the leg care apparatus according to an embodiment, it is possible to perform care on all the leg portions under the thigh. For example, a knee care part 240 may include at least one light emitting element 241 and at least one massage pad 242 to care the knee (see FIG. 6). It is already described that the foot bath is performed by the conduction and the convection even on the calf and the foot.

Thus, the leg care apparatus according to an embodiment may perform various foot bath functions and care functions for the body through various heating devices. Particularly, the user may perform the foot bath through all methods of radiation, conduction, and convection, which are capable of being expected in the foot bath. In this case, it is possible to provide the best function for the user who wants the hot fomentation.

Hereinafter, an effect of the leg care apparatus corresponding to the foot bath function of the strong hot fomentation will be described. The function of the strong hot fomentation may be applied more preferably in the case of the elderly.

First, a configuration of the knee care part 240 will be described.

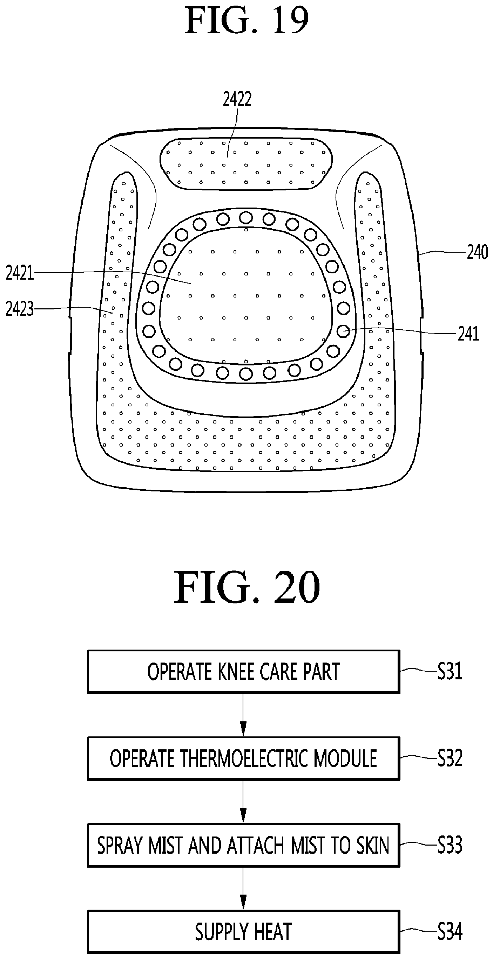

FIG. 19 is a view illustrating a configuration of the knee care part.

Referring to FIG. 19, the knee care part 240 includes at least one light emitting element 241 that irradiates infrared rays to the knee and applies heat, and at least one massage pad 242 that performs massage by pressing the periphery of the patella. The massage pad 242 may massage the knee through pressing and relaxation. The light emitting element 241 and the massage pad 242 may be attached to an inner surface of the knee care part 240.

Specifically, the massage pad 242 may include a central massage pad 2421 for massaging a portion below patella femoris, an upper massage pad 2422 disposed above the central message pad 2421, and a circumferential massage pad 2423 for massaging the peripheral portion of the patella except for the upper side. The massage pad 242 may relieve knee pain through continuous pressing and relaxation.

A plurality of light emitting elements 241 surrounds the peripheral portion of the central massage pad 2421. The light emitting element 241 may care the knee by irradiating a radiant heating source such as the infrared rays. the user may expect pain reduction and pain relief effects of the knee through the knee care part 240. The heat generated from the light emitting element 241 may be heated by directly heating the user's knee and also may provide radiant heat to the action space 500.

A method for controlling the leg care apparatus according to an embodiment, which performs the foot bath for the strong hot fomentation will be described.

FIG. 20 is a flowchart for explaining a method for controlling a leg care apparatus according to another embodiment.

Referring to FIG. 20, the knee care part 240 is operated (S31). Here, a light emitting element 241 may be operated to apply radiant heat by infrared rays to the inside of an action space 500. A knee care part 240 may not only care the user's knee inserted into the action space 500, but also radiantly heat an inner space of the action space 500 directly when the user's feet are not inserted into the action space 500.

The knee care part 240 may be operated or together with the knee care part 240, the thermoelectric modules 330 and 420 may be operated (S32). As the thermoelectric module is operated, it is possible to care the user's feet and calf inserted into the action space 500. In a state in which the user's leg is not inserted into the action space 500, the thermoelectric modules may directly transfer conductive and convective heat to an inner space of the action space 500. Alternatively, it may also transfer low radiant heat, but since the temperature is not high, the amount of heat may be significantly less than the conductive and convective heat.

The knee care part 240 and the thermoelectric modules 33 and 420 may provide heat to the action space 500 more quickly than other heating parts.

The knee care part 240 and the thermoelectric modules 330 and 420 may be operated to maintain a high-temperature atmosphere having a predetermined temperature or more inside the action space 500. Here, mist may be sprayed through the atomizer 130 (S33). Since the mist is in a state of receiving energy in the high-temperature action space, when covering the user's leg, the user's leg may be more quickly foot bathed.

The user may insert their leg into the action space 500 while the knee care part 240 and the thermoelectric modules 330 and 420 are operated. In this case, since the leg is inserted into the preheated action space, the foot bath function may be more quickly performed. In the case in which the mist is sprayed in advance, since the mist leaks to the outside, it is not preferable to be operated before the user's leg is inserted into the action space 500. This may be equally applied to the case of the blower 101.

Thereafter, as described above, at least one of a heating tool including a heating wire 1011 and the blower 101 may be operated to maintain the high-temperature mist. The mist may be maintained at a higher temperature. Here, the hot air current may contact the skin of the leg so that condensed water droplets are heated.

According to this embodiment, the user of which their leg is inserted into the action space 500 may quickly start the foot bath in a hot atmosphere.

According to this embodiment, the user may implement the foot bath function according to strong hot fomentation.

In the leg care apparatus according to an embodiment, a foot bath function is provided in a manner in which the leg care apparatus direct contacts the skin of the leg. For example, constituents illustrated as a foot contact pad 421 and a calf contact pad 331 may perform the foot bath function by transferring heat contacting a sole and calf of the leg.

The conductive heat transfer method of transferring heat directly contacting the skin does not require a separate medium for indirect heat transfer in the foot bath. For example, it is different from the foot bath that generally requires water as the medium. The conductive heat transfer method is suitable for use due to high thermal efficiency, rapid operation start, and convenient care.

In the method for implementing the conductive heat transfer method, there is a limitation of sudden temperature change, burn risk, and fire concern when using ohmic resistance heat that is generally used. In view of such a limitation, in the leg care apparatus according to an embodiment, a thermoelectric module may be used. However, the embodiment does not exclude the use of ohmic resistance heat and separately using a thermostat. Likewise, it does not exclude other methods of artificially creating a temperature atmosphere.

The thermoelectric module will be described in detail.

The thermoelectric module is an electric device in which a semiconductor is integrated, which generates current by flowing current and absorbs heat on the other side. In recent years, the thermoelectric module has improved in efficiency and is widely used in real life. The thermoelectric module may be a forced heat transfer device using a semiconductor, and there is no abrupt temperature change, and there is a difference depending on the material, but a temperature suitable for the foot bath of the human body may be easily obtained. The thermoelectric module may switch the heat generation-side and the heat absorption-side by reversing a current direction. Accordingly, in the leg care apparatus according to an embodiment, there is an effect that is carried out not only in the hot fomentation but also in the cold fomentation.

In the leg care apparatus according to an embodiment, the thermoelectric module 420 corresponding to the sole of the foot and the thermoelectric module 330 corresponding to the calf are provided. The thermoelectric modules 420 and 330 may contact the skin of the user through the foot contact pad 421 and the calf contact pad 331, respectively.

Since each of the contact pads 421 and 331 is made of a metal having high thermal conductivity, heat may rapidly spread over the entire surface of the contact pad, even if any part of the contact pad contacts the respective thermoelectric modules 420 and 330.. Accordingly, the foot bath function may be performed on all skin surfaces contacting the contact pads 421 and 331. The calf and the sole are elastic portions of the human body, and thus, a wide contact range may be performed more easily.

The thermoelectric module includes a surface contacting a contact pad to transmit a thermal atmosphere desired by the user and the other surface disposed to exhaust a thermal atmosphere opposite to that desired by the user. The other surface of the thermoelectric module may be exposed to the outside air outside the action space to perform natural or forced convection. According to an embodiment, in the thermoelectric module 420, the forced convection is performed by the heat exchange fan 423. This may create a stronger temperature atmosphere for the foot portion at which the main foot bath function is performed. The natural convection may be performed on the calf thermoelectric module 330. Of course, the forced convection may also be performed on the calf thermoelectric module 330.

Whether to provide a separate heat exchange on the other surface of the thermoelectric module may correspond to a temperature load required by the contact pad. For example, if a large amount of heat transfer is required for the thermoelectric module, it may be possible to provide a separate heat exchange such as the forced convection. Since the sole has a thick skin and a relatively hot temperature atmosphere, and the temperature load is large, the heat exchange fan 423 as described above may be provided. On the contrary, since the calf has a thin skin thickness, and a relatively small heat load, heat exchange may not be provided.

Each of the contact pads 421 and 331 may be made of a metallic material having high thermal conductivity. Since the contact pad is made of a metal such as stainless steel, the user may feel uncomfortable because the contact pad becomes cold when the temperature of the contact pad is not artificially controlled.

A method for controlling the leg care apparatus to improve such inconvenience according to an embodiment will be described.

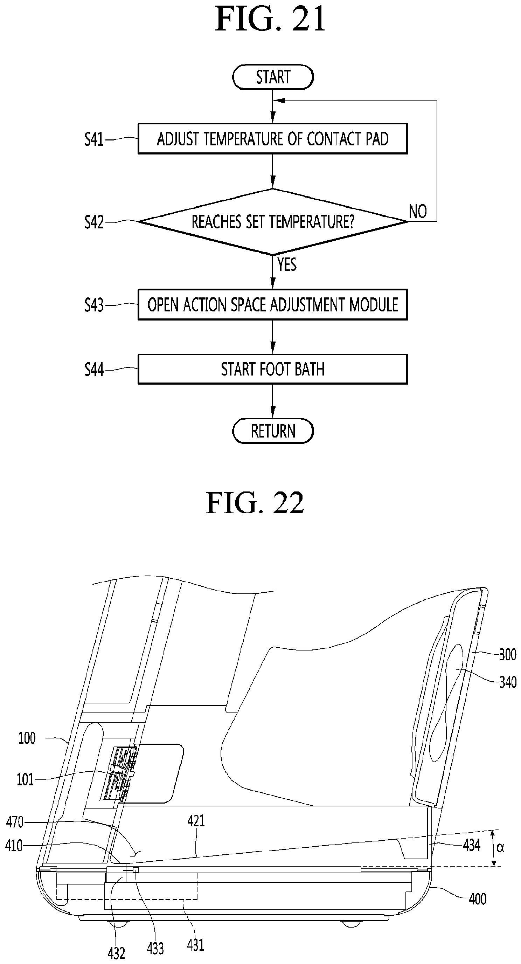

FIG. 21 is a flowchart for explaining a method for controlling a leg care apparatus according to another embodiment.

Referring to FIG. 21, when a user intends to have a foot bath in a conductive manner, first, a temperature of contact pads 421 and 331 is adjusted (S41). When current flows through thermoelectric modules 420 and 330, a temperature of one side of the thermoelectric modules 420 and 330 contacting the contact pads 421 and 331 may be adjusted. A temperature control of the contact pad may be performed until the temperature of the contact pad reaches a preset temperature.

A set temperature may be set differently from the foot bath temperature suitable for performing the foot bath. For example, the set temperature may be set lower than the foot bath temperature. This is done because the set temperature corresponds to a temperature at which the user's skin does not feel discomfort when contacting the contact pad.

When the temperature of each of the contact pads 421 and 331 reaches the set temperature (S42), an inlet may be opened using an action space adjustment module (S43).

In the method for controlling the leg care apparatus according to the embodiment, the user may select a display 270 to take the foot bath in the conduction manner. In this case, prior to opening the inlet by using the action space adjustment module, the contact pad may reach the set temperature first. As a result, the user may be prevented from discomfort in that the user's skin does not contact the contact pads 421 and 331 that have not been prepared.

The user may insert their leg into the action space 500 of which the inlet is opened, and the user may enjoy the conductive foot bath (S44).

According to the method for controlling of the leg care apparatus, the user may enjoy the foot bath by the conductive method more conveniently.

The leg care apparatus according to an embodiment may perform the foot bath on the sole. Here, the foot bath may be performed in the manner of conduction, radiation, and convection. In addition, the foot bath may be performed by dipping the feet in water. Hereinafter, a description of the leg care apparatus, which performs the foot bath by dipping the feet in the standing water, according to another embodiment will be given.

FIG. 22 is a schematic cross-sectional view that cuts a lower portion of the leg care apparatus in a front and rear direction according to another embodiment. In the another embodiment, water contained in the lower portion is different from the above-described embodiment, and in the description according to this embodiment, the description of the above-described embodiment is applied as it is, and the description thereof is omitted.

Referring to FIG. 22, at least a portion of the bottom frame 410 is formed to be inclined downward from the front to the rear. The floor frame 410 may be inclined at a predetermined inclination angle α toward the front side. An upper space of the bottom frame 410 may provide a water storage part 470 in which water may be stored. Since the water storage part 470 is provided to be inclined, a rear portion may be filled with water first, and a front portion may be filled with water later.

Side and rear sides of the water storage part 470 may provide a blocking wall by its own walls of the main body 100 and the bottom module 400. A front side of the water storage part 470 may provide a blocking wall by a water leakage prevention layer 434 in which the front surface part of the bottom module 400 protrudes to a predetermined height.

A rear portion of the water storage part 470 is provided with a drain pipe 432, and thus, the water may be drained. The drainage pipe 432 may be provided with a valve 433, and the water in the water storage part 470 may be drained through the drainage pipe 432 according to the control of the valve 433. One end of the drain pipe 432 may be aligned with the water tray 431 so that the water drained from the water storage part 470 is collected in the water tray 431. The water collected in the water tray 431 may be discharged by the user or automatically.

The drain pipe 432 may be provided at the lowest position in the inclined water storage part 470. The water storage part 470 may be drained through the drain pipe 432 without any water remaining. Of course, there may be residual water contacting the inner surface of the action space 500. However, the residual water may be evaporated by forced air blown from the blower 101. According to this, the inside of the action space 500 may be maintained in a clean state.

The water storage part 470 may adjust a height of each blocking wall to reach a height up to the ankle. The embodiment is not limited thereto and may reach a height up to the entire sole. This is done because the user's foot lying inside the action space may already be performed up to the foot.

The water stored in the water storage part 470 may be performed by the water condensed and dropped by mist supplied from an atomizer 130. The mist supplied from the atomizer 130 is condensed on the inner surface of the action space and the user's leg surface to drop down. The dropping condensed water is collected in the water storage part 470 to perform a foot bath function for the user's feet.

Since the water stored in the water storage part 470 is continuously heated by a heating source of the thermoelectric module 420, the dropping condensed water may be warmed again even when a temperature is low.

In the embodiment, the foot bath may be performed using water stored in the water storage part 470. To increase in function of the foot bath when the foot bath is performed on the foot, it is important to make the user's foot to make contact with the foot contact pad 421. A pair of foot contact pads 421 may be provided in a symmetrical pair to allow the user to make contact with both feet so as to receive hot or cold air.

Hereinafter, the foot contact pad will be described in detail.

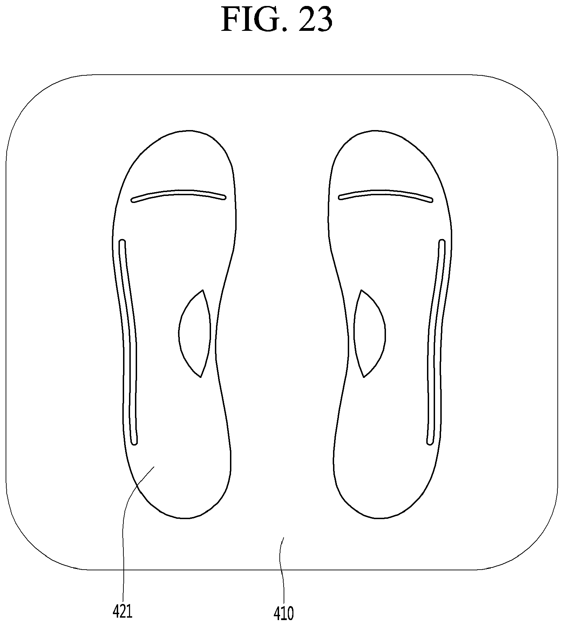

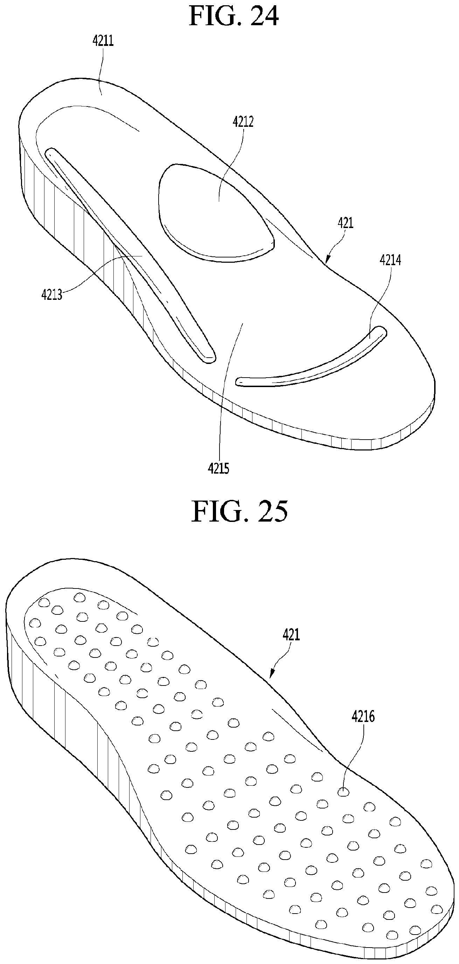

FIG. 23 is a top view of the leg care apparatus according to another embodiment, and FIG. 24 is a perspective view of a foot contact pad according to the another embodiment.

Referring to FIGS. 23 and 24, a foot may contact a foot contact pad 421 to transfer heat to the user's foot. A thermoelectric module 420 may be provided below the foot contact pad 421 to transmit hot or cold air to the foot contact pad 421.

The foot contact pad 421 may be provided with a foot contact surface 4215 contacting a sole and an edge correspondence part 4211 protruding smoothly from an outer circumference of the foot contact surface 4215 to contact an edge portion of the user's foot. Accordingly, the hot or cold air may be applied to an entire part of the user's foot.

The edge correspondence part 4211 may be provided only at a portion corresponding to a heel portion of the foot and a side portion of the foot and may be opened without being provided at a toe portion, i.e., at a rear side of the action space. Accordingly, condensed water condensed by mist from the leg may fall down to smoothly flow to a water storage part 470 along the foot and then be collected. To allow the condensed water to smoothly flow to the water storage part 470, like a bottom frame 410, the foot contact surface 4215 may also be inclined downward toward a rear side.

A protruding portion may be provided on the foot contact surface 4215 to smoothly transfer heat between the foot contact surface 4215 and the user's foot. Particularly, first, an inner arch correspondence part 4212 protruding from the foot contact surface 4215 at a portion corresponding to an inner arch of the user's foot, and second, an outer arch correspondence part lengthily protruding from a portion of the outer arch in a front and rear direction, and third, a toe recess part correspondence part 4414 corresponding to a distance between a sole and a toe may be provided.

According to the correspondence parts 4212, 4213, and 4214, contact between the foot contact surface 4215 and the sole of the foot may be facilitated. Accordingly, a foot bath function using high temperature water stored in the water storage part 470 and a foot bath function using conductive heat conducted through the foot contact pad 421 may be used together. As a result, an intensive foot bath effect may be obtained on the user's foot.

In the above description, the foot contact pad 421 performs the foot bath through heat, but is not limited thereto. For example, water at room temperature may accumulated in the water storage part 470, and cold air may be supplied through the foot contact pad 421 to perform cold fomentation.

FIG. 25 is a perspective view of the foot contact pad according to another embodiment.. In this embodiment, other parts are the same as those of FIGS. 23 and 24, and thus the description thereof will be omitted, and only the parts that are characteristically changed will be described.

Referring to FIG. 25, a plurality of protrusions 4216 are disposed on a top surface of the foot contact pad 421. The contact between the foot and the foot contact pad may increase by using elasticity of the foot of the user through the protrusions. The massage protrusion may also perform a massage function on the foot.

Another embodiment included in the spirit of the present disclosure is further described.

One of the upper module and the side module may not be provided. Thus, one of the upper module and the side module may be opened so that the user inserts their leg into an action space. In this case, there is a limitation that the user's inconvenience increases slightly, but the operation and action of the leg care apparatus is not impossible. Nevertheless, the embodiment in which both the upper module and the side module are provided is most preferable for the convenience of the user.

One of the foot contact pad and the calf contact pad may not be provided. Thus, even if one contact pad is used, heat and cold air may be transferred to the legs by other heat transfer part. In this case, there is a limitation that the user's inconvenience increases slightly, but the operation and action of the leg care apparatus is not impossible. Nevertheless, the embodiment in which both the leg contact pad and the calf contact pad are provided is most preferable for the convenience of the user.

Although the atomizer is described as being provided to the bottom module, the embodiment is not limited thereto, and thus, the atomizer may be provided below or above the main body. The foot bath due to conductive heating by the mist and thermoelectric module and the foot bath due to convection heating by the blower may be selectively performed to perform the foot bath.

According to the embodiment, the user may adjust the size and the like of the leg care apparatus to be suitable for their own body and conveniently operate the leg care apparatus.

According to the embodiment, since the leg car apparatus is safely used even in the high humidity environment, the risks of burns and electric shock may be reduced.

According to the embodiment, the use may conveniently move the leg care apparatus, and the leg care apparatus may be conveniently used in a narrow indoor space due to the compact size thereof.

According to the embodiment, the leg care apparatus may include a firm frame so as to be used for a long time without being damaged.

## Claims

1. A leg care apparatus comprising:
a main body (100) configured to provide an action space (500) to accommodate a leg; and
at least one of an atomizer (130) configured to provide mist to the action space (500) or a contact pad (331, 421) of which one surface is exposed to an inner surface of the action space (500) so as to care the leg placed in the action space (500) .

2. The leg care apparatus according to claim 1, wherein the contact pad (331, 421) is disposed on the inner surface of the action space (500) to correspond to at least one point of a sole or a calf.

3. The leg care apparatus according to claim 1 or 2, wherein another surface of the contact pad (331, 421) corresponds to a thermoelectric module to create a low or high temperature environment.

4. The leg care apparatus according to any one of claims 1 to 3, further comprising:
a bottom module (400) which provides at least a portion of a bottom surface of the action space (500) and in which components are accommodated therein; and
a water storage part (470) disposed at a top surface of the bottom module (400) to collect water.

5. The leg care apparatus according to claim 4, wherein a bottom surface of the water storage part (470) is gradually inclined downward from a front side to a rear side.

6. The leg care apparatus according to any one of claims 1 to 5, wherein the atomizer (130) comprises at least one of:
a room-temperature atomizer (1301) configured to spray room-temperature mist; and
a high-temperature atomizer (1302) configured to spray high-temperature mist.

7. The leg care apparatus according to claim 6, wherein the room-temperature atomizer (1301) is configured to supply fine water particles by using ultrasonic waves.

8. The leg care apparatus according to claim 6, wherein the high-temperature atomizer (1302) is operated in a tank heating manner in which water stored in a tank is heated.

9. The leg care apparatus according to any one of claims 1 to 8, further comprising:
an upper module (200) that is relatively movable with respect to the main body (100);
a water supply device (250) disposed at one portion of the upper module (200) to receive water to be supplied to the atomizer (130);
a water storage tank (10) configured to supply the water to the atomizer (130) and store the water received from the water supply device (250); and
a conduct pipe (255) configured to connect the water supply device (250) to the water storage tank,
wherein, preferably, the conduct pipe (255) is flexible.

10. The leg care apparatus according to any one of claims 1 to 9, further comprising a blower (101) configured to generate an air current within the action space (500).

11. The leg care apparatus according to claim 10, further comprising a heating wire (1011) configured to heat air blown from the blower (101).

12. The leg care apparatus according to any one of claims 1 to 11, further comprising a side module (300) configured to adjust a front and rear size of the action space (500) .

13. The leg care apparatus according to any one of claims 1 to 12, further comprising a knee care part (240a, 240b) disposed to correspond to a user's knee within the action space (500).

14. The leg care apparatus according to claim 13, wherein the knee care part (240a, 240b) comprises:
at least one light emitting element (241); and
a massage pad (242) configured to press or release a peripheral portion of the knee.

15. A method for controlling a leg care apparatus, the method comprising:
adjusting a size of an action space (500) to fit a user's leg accommodated in an action space (500); and
supplying fine water particles to cover the user's leg and controlling a temperature state of a contact pad (331, 421) for adjusting a contact temperature of a user's skin so as to care the user's leg,
wherein, preferably, the fine water particles are heated by hot air current,
wherein, preferably, the contact part (331, 421) is controlled to be cooled so that a cold area is formed on an inner surface of the action space (500), and
wherein, preferably, the contact part (331, 421) corresponds to any one place of a user's calf and sole.
